(19) **Europäisches Patentamt**
**European Patent Office**
**Office européen des brevets**

(11) **EP 1 861 494 B1**

(12) **EUROPEAN PATENT SPECIFICATION**

(45) Date of publication and mention
of the grant of the patent:
**08.11.2017 Bulletin 2017/45**

(51) Int Cl.:
*C12N 9/34* (2006.01)    *C12N 15/62* (2006.01)
*C12N 15/63* (2006.01)   *A61K 8/66* (2006.01)
*C07K 1/22* (2006.01)    *C12Q 1/68* (2006.01)
*A61Q 11/00* (2006.01)   *C12Q 1/40* (2006.01)

(21) Application number: **06705712.5**

(22) Date of filing: **02.03.2006**

(86) International application number:
**PCT/CN2006/000305**

(87) International publication number:
**WO 2006/092099 (08.09.2006 Gazette 2006/36)**

(54) **RECOMBINANT PROTEIN COMPRISING STARCH BINDING DOMAIN AND USE THEREOF**

REKOMBINANTES PROTEIN MIT STÄRKEBINDUNGSDOMÄNE UND VERWENDUNG DAVON

PROTÉINE RECOMBINANTE COMPORTANT UN DOMAINE DE LIAISON À L'AMIDON ET SON UTILISATION

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.03.2005 US 70271**

(43) Date of publication of application:
**05.12.2007 Bulletin 2007/49**

(73) Proprietor: **Simpson Biotech Co., Ltd.**
**Taoyuan Country 333 (TW)**

(72) Inventors:
• **Chang, Margarete Dah-Tsyr**
**Taiwan (CN)**
• **Sheu, Chia-Chin**
**Taoyuan County,**
**Taiwan 333 (CN)**

(74) Representative: **Lang, Christian et al**
**LangPatent Anwaltskanzlei**
**IP Law Firm**
**Rosenheimer Straße 139**
**81671 München (DE)**

(56) References cited:
EP-A1- 0 186 066      WO-A1-98/16190
WO-A1-99/15636       WO-A2-00/77165
WO-A2-2005/014779    US-B1- 6 297 359

• CHEN ET AL: "Adsorption to starch of a beta-galactosidase fusion protein containing the starch-binding region of Aspergillus glucoamylase.", GENE, vol. 99, no. 1, 1 March 1991 (1991-03-01), pages 121-126, XP55007309, ISSN: 0378-1119
• TERPE K: "Overview of tag protein fusions: from molecular and biochemical fundamentals to commercial systems", APPLIED MICROBIOLOGY AND BIOTECHNOLOGY, SPRINGER VERLAG, BERLIN, DE, vol. 60, no. 5, 1 January 2003 (2003-01-01), pages 523-533, XP002298417, ISSN: 0175-7598
• PALDI TZUR ET AL: "Glucoamylase starch-binding domain of Aspergillus niger B1: Molecular cloning and functional characterization", BIOCHEMICAL JOURNAL, THE BIOCHEMICAL SOCIETY, LONDON, GB, vol. 372, no. 3, 15 June 2003 (2003-06-15) , pages 905-910, XP002496200, ISSN: 0264-6021, DOI: 10.1042/BJ20021527
• YU-WEN HUA ET AL: "Fusion of Bacillus stearothermophilus leucine aminopeptidase II with the raw-starch-binding domain of Bacillus sp. strain TS-23 alpha-amylase generates a chimeric enzyme with enhanced thermostability and catalytic activity", JOURNAL OF INDUSTRIAL MICROBIOLOGY & BIOTECHNOLOGY, vol. 31, no. 6, 1 July 2004 (2004-07-01), pages 273-277, XP55008219, ISSN: 1367-5435, DOI: 10.1007/s10295-004-0146-5

- HSIEN-BIN HUANG ET AL: "Construction and one-step purification of Bacillus kaustophilus leucine aminopeptidase fused to the starch-binding domain of Bacillus sp. strain TS-23 [alpha]-amylase", BIOPROCESS AND BIOSYSTEMS ENGINEERING, SPRINGER, BERLIN, DE, vol. 27, no. 6, 1 October 2005 (2005-10-01), pages 389-398, XP019347358, ISSN: 1615-7605, DOI: 10.1007/S00449-005-0001-8
- JANECEK S. ET AL.: 'The evolution of starch-binding domain' FEBS LETTERS vol. 456, 31 December 1999, pages 119 - 125, XP004260050
- ASHIKARI T. ET AL: 'RHIZOPUS RAW-STARCH-DEGRADING GLUCOAMYLASE ITS CLONING AND EXPRESSION IN YEAST SACCHAROMYCES-CEREVISIAE' AGRICULTURAL AND BIOLOGICAL CHEMISTRY vol. 50, no. 4, 1986, pages 957 - 964, XP008120586
- Yoshikazu Tanaka ET AL: "Comparison of amino acid sequences of three glucoamylases and their structure-function relationships.", Agricultural and Biological Chemistry, vol. 50, no. 4, 1 January 1986 (1986-01-01), pages 965-969, XP055170973, ISSN: 0002-1369, DOI: 10.1271/bbb1961.50.965
- CHOU WEI-I ET AL: "The family 21 carbohydrate-binding module of glucoamylase from Rhizopus oryzae consists of two sites playing distinct roles in ligand binding", BIOCHEMICAL JOURNAL, PORTLAND PRESS LTD, GB, vol. 396, no. Part 3, 1 June 2006 (2006-06-01), pages 469-477, XP002557688, ISSN: 0264-6021, DOI: 10.1042/BJ20051982 [retrieved on 2002-03-02]

Remarks:

The file contains technical information submitted after the application was filed and not included in this specification

Description

## FIELD OF THE INVENTION

This invention relates to the starch binding domain (SBD) consisting of the amino acid sequence of SEQ ID No. 1. A recombinant protein comprising the SBD can be produced and purified by using the SBD as a tag. This invention further relates to a novel method and a kit to purify the recombinant protein and novel applications of the SBD and the recombinant protein. Also disclosed are a fibril comprising starch binding domain and a method of identifying a ligand binding site of a starch binding domain of a candidate amyloytic enzyme.

## BACKGROUND OF THE INVENTION

Production of proteins by expression in microbial systems has become a significant source of high value, medically important proteins. Purification and recovery of recombinant proteins are major considerations in the design of a fermentation process. While traditional methods of protein purification can be used to isolate a product, improved methods include the use of recombinant proteins. Recombinant proteins can be purified by affinity column chromatography, the desired component of the recombinant protein being purified by virtue of its covalent attachment to a polypeptide, which binds to an affinity matrix.

Certain systems exist for isolating proteins by the principle of affinity column chromatography.

U.S. Pat. No. 5643758 describes a system comprising maltose-binding protein (MBP). A cloned gene is inserted into a pMAL vector down-stream from the malE gene, which encodes MBP. The vector is transformed to a host cell, then the recombinant protein can express in the host cell. The cell lysate or media fraction is loaded to a column containing affinity matrix amylose and washed several times, then using a large amount of maltose to elute the recombinant protein.

U.S. Pat. No. 5202247 describes a system comprising cellulose-binding domain. A cellulose column can be used to purify the recombinant protein that contains cellulose-binding domain. The cell lysate or media fraction is loaded to the column and washed. The interaction between cellulose-binding domain and cellulose appears to be driven by hydrophobic interaction at neutral pH. The general method for elution used low polarity solvents such as ethlylene glycol, prior to removal of the low polarity solvents by dialysis and filtration.

A chitin-binding domain and an inducible-splicing linker region can be fused in the *C*-terminus or *N*-terminus of a target protein. The cell lysate or media fraction is loaded to the column and washed. The chitin-binding domain binds to the chitin column to immobilize the recombinant protein. In the presence of thiols such as DTT or cysteine, the linker region undergoes specific self-cleavage to release the target protein from the chitin-bound chitin-binding domain.

These current protein purification systems have some disadvantages. The purification processes are inconvenient and laborious. The columns used in purification are expensive. Limitations for protein purification of these systems include unable to isolate the recombinant protein in certain conditions such as EDTA-containing samples as well as the current protein tags being used are relatively large as compared to the target protein bigger than that of this invention.

EP 0 186 066 A1 discloses glucoamylases of Rhizopus Oryzae comprising the sequences of SEQ ID NO. 2 and 3, respectively. WO 00/77165 A2 discloses a recombinant protein having a starch binding domain including SEQ ID NO. 2.

## SUMMARY OF THE INVENTION

The above-mentioned object is solved by the starch binding domain consisting of the amino acid sequence of SEQ ID No. 1 Further developments of the invention are subject matter of dependant claims.

This invention also provides a recombinant protein comprising a polypeptide and the starch-binding domain of the invention. This invention also provides an expression vector comprises a gene encoding the starch binding domain of the invention. This invention further provides a method for purifying a recombinant protein comprising a polypeptide and the starch binding domain of the invention from a biological liquid. This invention further provides a kit for purifying a recombinant protein comprising an expression vector used to express the recombinant protein.

## BRIEF DESCRIPTION OF THE DRAWINGS

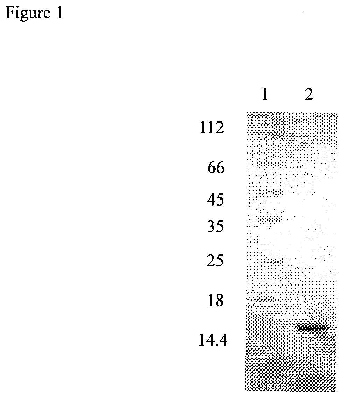

Figure 1 illustrates purification of SBD-6H using starch. The purified SBD-6H is subjected to SDS/PAGE (15% gel) and the ge1 is stained with Coomassie Brilliant Blue R-250 for protein detection. Lane 1, molecular mass standards (molecular masses indicated at the left); lane 2, Two microgram of SBD-6H that is purified on corn starch.

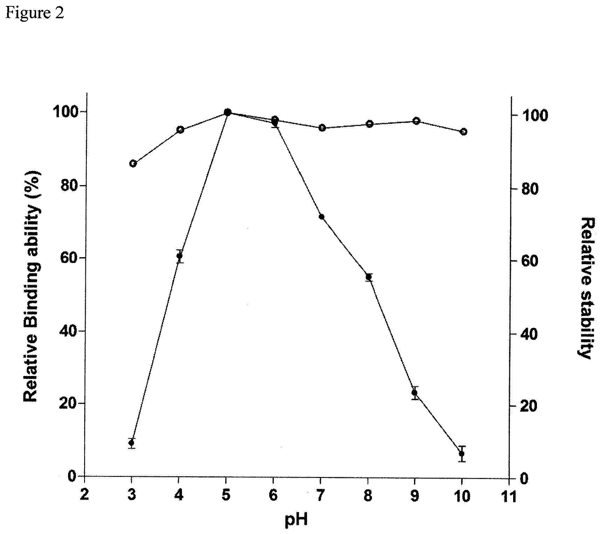

Figure 2 illustrates effects of pH on binding ability and stability. The effects on binding ability (●) and stability (○) of the SBD-6H at varying pH are measured as described in the Experimental section. The relative binding ability of SBD-6H assayed in pH 5 is normalized as 100%.

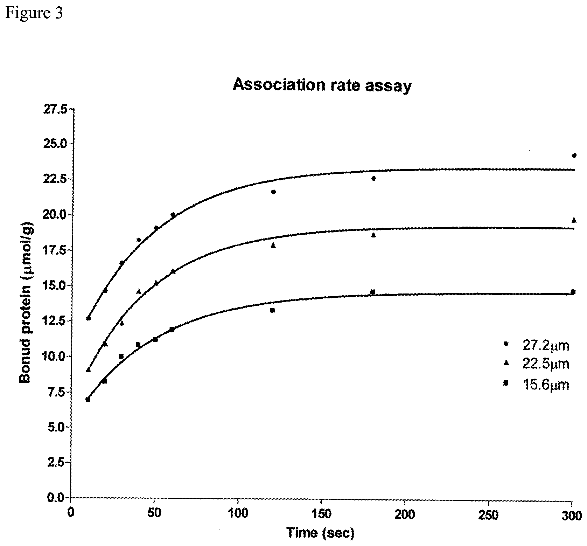

Figure 3 illustrates adsorption rate of SBD-6H with insoluble starch. The association rate of SBD is analyzed by applying SBD-6H at different concentrations (■, 15.6 $\mu$M; ▲, 22.5 $\mu$M; ●, 27.2 $\mu$M) to corn starch for 5 h. Bound protein is calculated from the difference between the initial and unbound protein concentrations at different time points to determine the association rate.

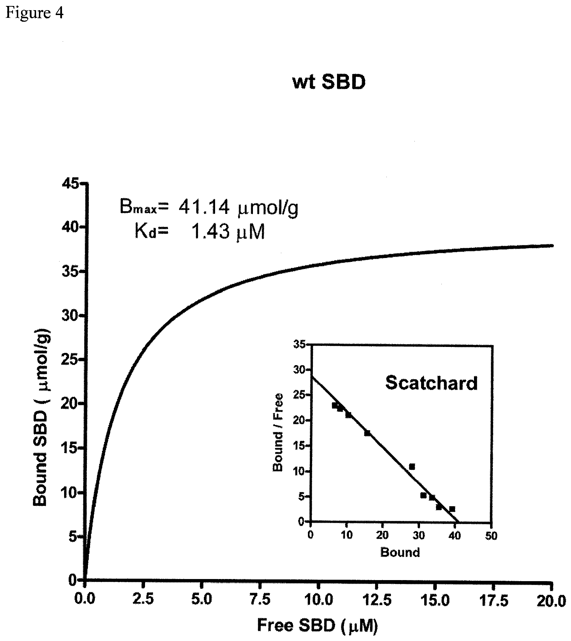

Figure 4 illustrates $K_d$ and $B_{max}$ determination of the SBD-starch interaction. Starch binding assays are performed with granule corn starch at different protein concentrations. The $K_d$ and $B_{max}$ are determined by fitting to the non-linear regression of the binding isotherms for one binding site saturation binding.

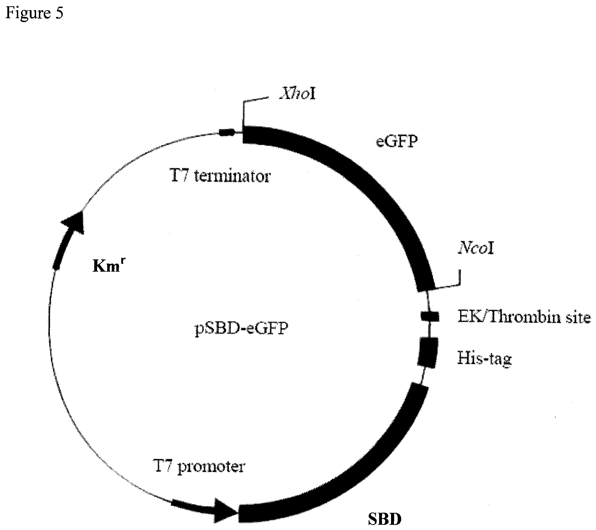

Figure 5 shows construction of SBD-tagged fusion proteins. eGFP: gene encoding green fluorescent protein; SBD: starch binding domain; Km[r]: kanamycin resistance gene.

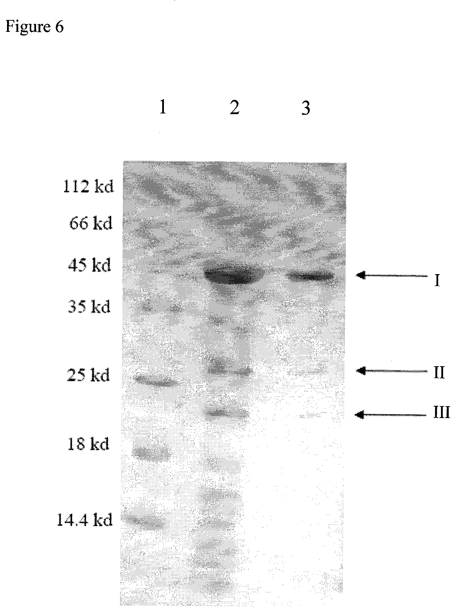

Figure 6 illustrates purification of SBD-tagged fusion proteins using starch. The purified fusion protein is subjected to SDS/PAGE (15% gel) and the gel is stained with Coomassie Brilliant Blue R-250 for protein detection. Lane 1, molecular mass standards (molecular masses indicated at the left), lane 2, soluble fraction, and lane 3, elution. Arrowhead I: SBD-eGFP, arrowhead II: eGFP, and arrowhead III:SBD.

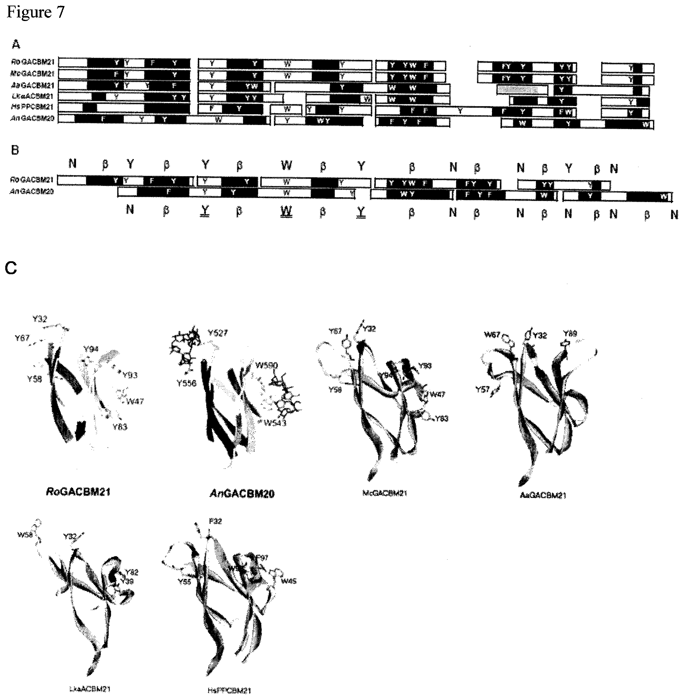

Figure 7 illustrates comparison of the structure-based multiple sequence alignment of CBM20 and CBM21. (A) Structure-based multiple sequence alignment with SBDs of *R. oryzae* (*Ro*GACBM21), *M. circinelloides* (*Mc*GACBM21), *A. adeninivorans* (*An*GACBM21), *Lipomyces kononenkoae $\alpha$-amylase* (*Lk$\alpha$*ACBM21), *Homo sapiens* protein phosphotase (*Hs*PPCBM21), and *A. niger* (AnGACBM20). The white, black and gray segments represent the predicted secondary structure elements loop, $\beta$-strand and $\alpha$-helix, respectively. *Y, W* and *F* in *black* indicate tyrosine, tryptophan and phenylalanine in the loop region, respectively; *Y, W* and *F* in *white* represent the aromatic residues in $\beta$-strands. (B) Structure-based sequence alignment using the PSSC algorithm with the schematized sequences of *Ro*GACBM21 and *An*GACBM20 marked above and below the figure, respectively. *$\beta$* represents a $\beta$-strand; *N* indicates that there is no aromatic residue in the loop region. The key ligand binding residues in *An*GACBM20 are denoted by a *double underline.* (C) The simulated three dimensional structures and putative ligand binding sites of *R. oryzae* (*Ro*GACBM21), *M. circinelloides* (*Mc*GACBM21), *A. adeninivorans* (*An*GACBM21), *Lipomyces kononenkoae $\alpha$-amylase* (*Lk$\alpha$*ACBM21), *Homo sapiens* protein phosphotase (*Hs*PPCBM21) with the known structure and ligand binding sites of *A. niger* (AnGACBM20) as a template according to the secondary structure and topology mapping method of the present invention.

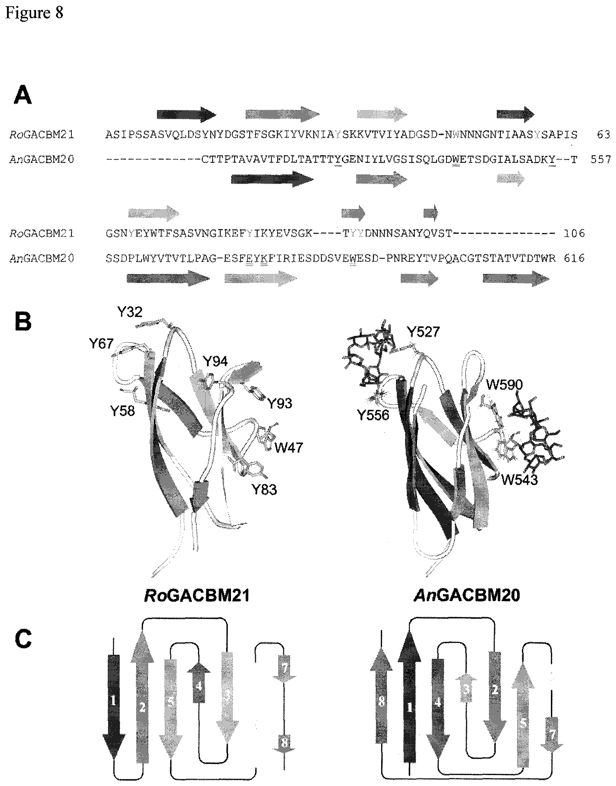

Figure 8 illustrates molecular modeling of *Ro*GACBM21 using structure-based sequence alignment. (A) The locations of the $\beta$-strands in *Ro*GACBM21 and *An*GACBM20, represented as grey level-coded arrows, are indicated above and below the sequences, respectively. The ligand binding sites in *An*GACBM20 are *underlined* (binding site I, _ ☐ binding site ..II ≡). The aromatic residues predicted to be involved in carbohydrate binding in *Ro*GACBM21 are labeled in grey. (B) The modeling structure of *Ro*GACBM21 (left panel) and the template structure of *An*GACBM20 (right panel, Protein Data Bank code 1AC0) are shown as ribbon diagrams. The polypeptide sequence from Ala[1] to Ser[13] forming the first $\beta$-strand of *Ro*GACBM21 is not shown in the model. The ligands ($\beta$CD) and the side chains of the characterized ligand binding sites in *An*GACBM20 as well as the potential binding sites of *Ro*GACBM21 are also depicted. (C) The topology of secondary structural directionality and organization of *Ro*GACBM21 and *An*GACBM20 are shown with grey level-coded arrows corresponding to those in *A* and *B.*

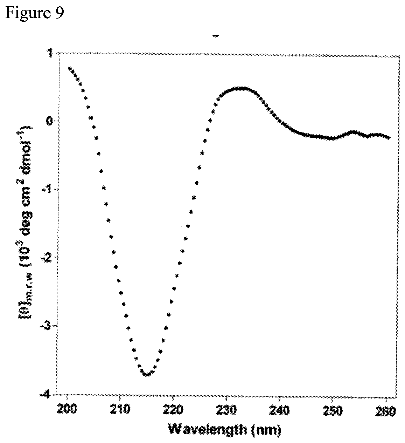

Figure 9 illustrates determination of the secondary structure of *Ro*GACBM21 by far-UV CD. The CD spectrum of the native *Ro*GACBM21 (*closed circles*) is performed in 50 mM sodium acetate (pH5.5) at 25 °C.

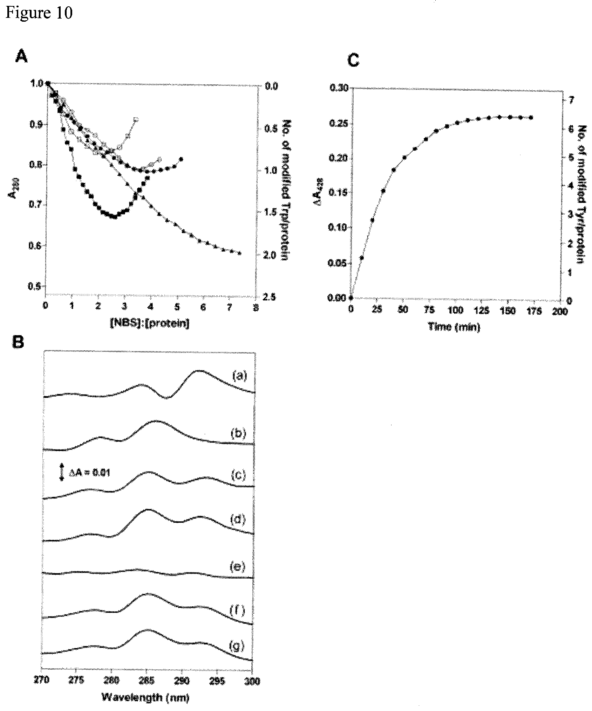

Figure 10 illustrates chemical modifications and UV difference spectra of *Ro*GACBM21 derivatives. (A) Native wild type *Ro*GACBM21 in the absence (*closed squares*) or presence (*open squares*) of 500 $\mu$M $\beta$CD; denatured wild type *Ro*GACBM21 (*closed triangle*) and native W47A in the absence (*closed circles*) or presence (*open circles*) of 500 $\mu$M $\beta$CD were titrated with NBS. The data were normalized to adjust the original absorbance of each reaction to 1.0. (B) Difference spectra of *N*-acetyltryptophan (50 $\mu$M) (*a*) and *N*-acetyltyrosine (100 $\mu$M) (b) perturbed with 20% DMSO were used for comparison. Difference spectra of wild type recombinant *Ro*GACBM21 (*c*), Y32A (*d*), W47A (*e*), Y16A (f), and Y86A (g) perturbed with $\beta$CD were normalized to an equivalent protein concentration. (C) Wild-type *Ro*GACBM21 (10μM, in 100 mM Tris-HCl, pH 8.0) was titrated with 100-fold excess TNM over protein at 25°C.

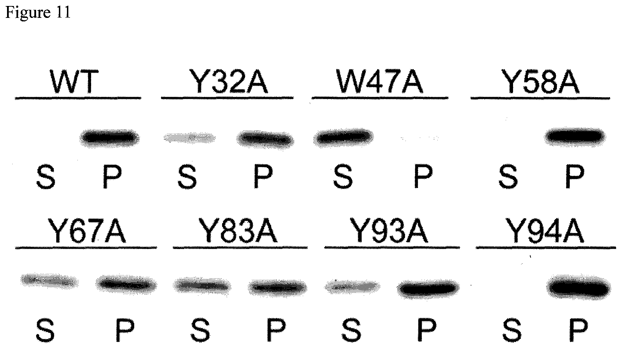

Figure 11 illustrates qualitative binding of *Ro*GACBM21 derivatives to insoluble starch, as analyzed by SDS-PAGE. Purified wild type and Y16A, Y32A, W47A, Y58A, Y67A, Y83A, Y86A, Y93A and Y94A mutants of *Ro*GACBM21 were incubated with insoluble starch, and than pelleted by centrifugation. Lane S represents protein not bound to insoluble polysaccharide; lane *P* contains protein bound to insoluble polysaccharide.

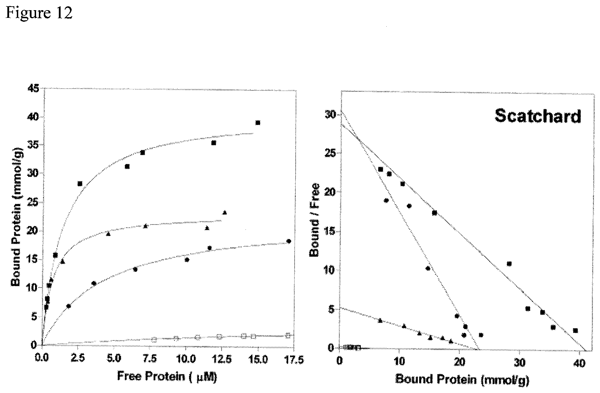

Figure 12 illustrates binding of *Ro*GACBM21 derivatives to insoluble starch, as analyzed by depletion isotherms. Starch-binding assays of *Ro*GACBM21 derivatives were performed with insoluble corn starch at different protein concentrations. Binding isotherm *(left panel)* and Scatchard analysis (*right panel*) of wild-type *Ro*GACBM21 (*closed squares*), Y32A (*closed triangle*), W47A (*closed circles*), and Y32A/W47A (*open squares*)

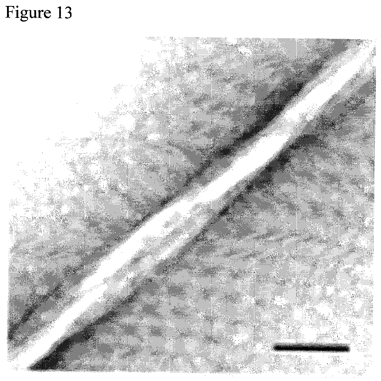

Figure 13 illustrates the fibril formed by starch binding domain under electron microscope.

## DETAILED DESCRIPTION OF THE INVENTION

The present invention is provided with a specially high affinity and strong binding ability of a starch binding domain (SBD) of an enzyme glucoamylase from *Rhizopus oryzae.* The glucoamylase (1,4-α-D-glucan glucohydrolase, EC 3.2.1.3) is a multidomain exo-acting glycoside hydrolase that catalyzes the release of β-D-glucose from the non-reducing ends of starch and related substrates. Glucoamylase is a modular protein comprising a catalytic domain (CD), and a starch binding domain (SBD), categorized to carbohydrate-binding modules (CBMs). The two independent domains are connected with an *O*-glycosylated linker (for detailed information, see URL http://afmb.cnrs-mrs.fr/CAZY/index.html; Boraston et al. Biochem. J. (2004) 382: 769-781; Fang et al. Protein Engineering (1998) 11:119-126; Sauer et al. Biochemistry (2001) 40: 9336-9346).

The CBMs mediate the interaction of glycoside hydrolases with substrates by concentrating the catalytic domains on the surface of insoluble polysaccharides (Bolam et al. Biochem. J. (1998) 331:775-781). The significant decreases in the activity of enzymes on insoluble but not soluble polysaccharides are observed by both proteolytic excision and truncation of CBMs from glycoside hydrolases (Charonck et al. Biochemistry (2000) 39:5013-5021; Ali et al. Biosci. Biotechnol. Biochem. (2001) 65:41-47). Different from all C-terminal SBDs belonging to the CBMs family 20, the *N*-terminal SBDs of glucoamylases are classified as members of CBM family 21. At present, glucoamylases from *Rhizopus oryzae, Arxula adeninivorans* and *Mucor circinelloides* have the CBM family 21 starch binding domain located at the *N*-termini (Houghton-Larsen et al. Appl. Microbiol. Biotechnol. (2003) 62: 210-217).

Comparison of the amino acid sequences of SBDs from these species and *A. niger* SBD shows that the homology between *R. oryzae* SBD and that of *A. adeninivorans, M. circinelloides,* and *Aspergillus niger* is 35.4%, 67.3% and 26.1%, respectively. The difference of the protein sequences between CBM 20 and CBM 21 families implies that these SBDs are distinct in biological and biochemical functions.

The polypeptide of the SBD can be obtainable from CBM20, CBM21, CBM25, CBM26, CBM34 and CBM41, preferably from CBM20 or CBM21, more preferably from starch binding domain of glucoamylase from CBM21, further more preferably from *Rhizopus spp,* and most preferably from *Rhizopus oryzae.* The amino acid sequences of SBDs obtainable from Simpson strain, wild type strain and a strain in U.S. Pat. No. 4863864 as shown in SEQ ID Nos. 1, 2, and 3 are different from each other. The SBDs of *Rhizopus oryzae* including allelic variation and derivatives have high affinity with starch and express strong starch-binding ability as compared with known $S_{BDS}$. The SBD of the invention consists of the amino acid sequence of SEQ ID No.1. The DNA molecule encoding SBD is amplified by PCR. The PCR product is cloned into an expression vector and transformed into a host cell. Transformed host cells are induced to express SBD. Cell lysate is harvested and the supernatant is applied directly to the affinity matrix, then washed and eluted. The elution buffer can be salt, sugar, and/or acidic or alkaline. The SBD of *Rhizopus spp.* is first characterized to be stable in a wide range of pH and dissociate with starch in acidic or alkaline environments. It is first identified that the dissociation constant ($K_d$) value of SBD in *Rhizopus spp* is lower than that of known species such as *Aspergillus niger,* and the starch binding ability of SBD in *Rhizopus spp* is much higher than that of known species such as *Aspergillus niger.* The dissociation constant ($K_d$) value of SBDs in *Rhizopus* to granular corn starch is 0.5 ~2. 29 μM. The more preferred aspect of the dissociation constant ($K_d$) value is 1.0 ~2. 0 μM. The further preferred aspect of the dissociation constant ($K_d$) value is 1.3~1. 6 μM. The most preferred aspect of the dissociation constant ($K_d$) value is 1.43 μM. The SBDs have active sites on aromatic group of amino acid residues 32, 47, 67, 83 and 93 of the sequence for carbohydrate-binding, wherein the amino acid residues are tyrosine or/ and tryptophan. In a preferred aspect, the active sites are residue 32 tyrosine, residue 47 tryptophan, residue 83 tyrosine and residue 93 tyrosine. In a more preferred aspect, the active site is residue 47 tryptophan and residue 32 tyrosine. The active sites consist of aromatic residues identified by progressive secondary structure correlation and topological mapping.

This invention also provides a recombinant protein comprising the SBD of this invention and a polypeptide of interest. A gene encoding a polypeptide of interest is cloned into the SBD-expression vector as previously described. The cloning site is neighboring the *sbd* gene, either upstream or downstream of *sbd* gene. The SBD can be linked with the polypeptide in *N*-terminus or in C-terminus. This fusion gene expression vector can be transformed or transfected into a host cell, including bacteria, yeast, fungi, mammalian cell, or insect cell. The recombinant protein can be expressed in the transformed or transfected cell. Thus, this recombinant protein according to this invention can be purified by using starch as affinity matrix, via the association between SBD and starch.

The affinity matrix used in recombinant protein purification can be a component recognized by SBD. The SBD according to this invention can bind the glucose-glucose linkage structure, both $\alpha$-1,4- and $\alpha$-1,6-linkage. By this characteristic, the affinity matrix can be a component containing the formula:

$$(X-X)_n$$

X means glucose molecule, the linkage between glucose and glucose is $\alpha$-1,4- linkage or $\alpha$-1,6-linkage and n is 1 or more than 1. One component containing the former structure in any part structure such as main chain, side chain, or modified residue can be selected to be affinity matrix. For example, starch, mannose, dextran, or glycogen can be affinity matrix.

This invention provides a recombinant protein purification method by using the starch binding domain of the invention and an affinity matrix. The method comprises (a) applying the biological liquid containing the recombinant protein directly to an affinity matrix; (b) eluting the recombinant protein by elution buffer; and (c) dialyzing the eluent containing the recombinant protein. This invention can be used to increase the production, activity, stability or solubility of recombinant proteins and polypeptides. This invention is suitable for large-scale protein purification with wide range of optimal pH. The yield of the purified recombinant protein is high with sufficient purity (greater than 95%). The advantages of this invention also includes that various elution buffers and affinity matrices can be chosen and be available commercially, the former includes sugar, salt, and/or pH and the latter includes starch, mannose, dextran, or glycogen. Further, SBD is a smaller tag than commonly used fusion protein tags including glutathione S-tansferase (GST), MBP, thioredoxin (Trx), or Nus, and this system is appropriated to purify particular samples comprising EDTA-, EGTA-, or DTT-containing sample.

This invention provides a kit to purify a recombinant protein and applications thereof. The kit comprises an expression vector to express the recombinant protein according to this invention. The kit further comprises an affinity matrix and an elution buffer. The affinity matrix can bind SBD to isolate the recombinant protein. The elution buffer is used to separate the association between SBD and affinity matrix.

The polypeptide of interest as previously described may be an antibody, an antigen, a therapeutic compound, an enzyme, or a protein. Via the kit provided by this invention, these products can be over-produced and purified rapidly. The applications of these products are described as follows.

The polypeptide of interest may be an antibody or a therapeutic compound, which targets and allows damage/destruction or detection of the pathogen. This invention allows the antibody or the therapeutic compound easily purified by using starch and can be further isolated by cleaving the SBD region. The purified product can be used for therapy or detection of pathogens.

The polypeptide of interest may be an antigen or an antigenic compound, which can induce immune responses. This fusion protein can bind to an affinity matrix such as starch. The fusion protein-bound starch can be supplied as source of foods. When eating the fusion protein-bound starch, users will gain vaccination effect. The fusion protein-bounded starch can be an oral vaccine.

The SBD of this invention can be applied for an oral care composition. The oral care composition comprises the SBD of the invention, wherein said composition is selected from the group consisting of a toothpaste, dental cream, gel or tooth powder, odontic, mouthish, pre- or post brushing rinse formulation, chewing gum, lozenge, and candy. The oral care composition further comprises a fusion product between one or more SBDs and an enzyme selected from the group consisting of oxidases, peroxidases, proteases, lipases, glycosidases, lipases, esterases, deaminases, ureases and polysaccharide hydrolases. This oral care product can digest oral polysacchride and prevent formation of dental caries.

The SBD of this invention can be further applied for a method for sorting carbohydrate-containing molecules by using different $K_d$ values of carbohydrate binding domains. The carbohydrate-containing molecules may be a mixture of various glycoproteins, and the carbohydrates may be monosacchride, disacchride, or polysacchride. For example, the users can sort glycoproteins by using starch-binding domains (SBDs) as a set of separator; maybe a column; with different $K_d$ values to generate a series of matrices with gradient ligand binding abilties. The SBDs may be obtainable from *Rhizopus spp* including wild type and mutants. The SBDs may be obtainable from the species which is known to produce SBDs such as CBM family 21 and CBM family 20. These known species include *Arxula, Lipomyces, Aspergillus, Bacillus, Clostridium, Cryptococcus, Fusarium, Geobacillus, Neurospora, Pseudomonas, Streptomyces, Thielavia,* and *Thermoanaerobacter.*

Loading the sample into the set of separator, the SBD-bound molecules are kept in the column and the unbound molecules are excluded. Thus, the sample can be sorted into SBD-bound and unbound groups. Collecting unbound molecules in one container, and eluting SBD-bound molecules in another container, both can be further sorted. The further sorting methods may be based on chemical, physical or biological properties including liquid column chromatographic analysis, mass spectrometric analysis, lectin immunosensor techniques, two-dimensional gel electrophoresis, enzymatical techniques, and chemical derivatizations. The SBD of this invention can be applied for providing a fibril comprising starch binding domain, which monomers interact with each other by beta-sheet interactions. The fibril can be in the form of a tubular structure. The fibril is applied to carbohydrate separation and drug delivery system.

The SBD of this invention can be applied for a method of identifying a ligand binding site of a starch binding domain of a candidate amyloytic enzyme comprising (a) making structure-based multiple sequence alignment and aromatic residues of both the candidate enzyme and a known amylolytic enzyme including a known ligand binding site, (b) comparing topology of the sequence alignment between the known enzyme and the candidate enzyme according to progressive secondary structure correlation algorithm (PSSC), and (c) correlating the known ligand binding site of the known enzyme to residue located at the correspondent positions in the candidate enzyme.

To identify the function of the resultant residue of the candidate enzyme, the method further comprises the measure of site-directed mutagenesis, chemical modification, UV difference spectroscopy or qualitative and quantitative binding assays.

The term "ligands" used herein is not limited but is selected from the group consisting of starch, cyclodextrin, maltoheptaose, maltohexaose and maltopentaose.

The candidate enzyme is selected from the group consisting of CBM20, CBM21, CBM25, CBM26, CBM34 and CBM41. The preferred enzyme is selected from CBM20 and CBM21. The more preferred enzyme is obtained from fungal genus *Rhizopus.* The most preferred enzyme is obtained from *Rhizopus oryze.*

The candidate enzyme is not limited but is glucoamylase, α-amylase, β-amylase, acarviose transferase, glucosyltransferase, glucanotransferase, cyclodextrin glucosyltransferase, maltopentaobydrulase, maltotetraohydrolase, maltogenic α-amylase, amylopullulanase, α-glucan water dikinase, genethonin-1, laforin, degreenig enhanced protein or protein phosphatase.

The known enzyme for use in predicting the ligand binding site of the candiadate enzyme is selected from CBM20, CBM25, CBM26 or CBM34. Preferably, the known enzyme is selected from CBM20 or CBM34. More preferably, the known enzyme is selected from *Aspergillus niger.*

In the method, the progressive secondary structure correlation (PSSC) algorithm comprises three steps: (*1*) obtaining multiple secondary structures using existing prediction systems to assign positions and boundaries of α-helices and β-strands, (*2*) transforming each of the predicted secondary structures into a new sequence representation by symbolizing the α-helices and β-strands as well as labeling selected aromatic residues, Y, W, and F, predefined as constrained residues, and (*3*) performing progressive correlation operations on one protein sequence with a resolved three-dimensional structure as the template.

In particular, the scores from all possible alignment sets between each of the predicted secondary structure and a known template structure is calculated. The one with the highest value represents the best alignment candidate in which the key residues corresponding to those in the template structure can be identified. The correlation operations are performed to provide the best alignment with respect to the location of secondary structures and constrained aromatic residues.

In the method, the topology is an immunoglobulin-like topology (CATH code 2.60.40).

EXAMPLE

The following examples are offered by way of illustration and not by way of limitation.

EXAMPLE 1

(A) CONSTRUCTION OF SBD-6H

The gene encoding glucoamylase SBD from residues 26-131 was amplified by polymerase chain reaction (PCR) using the forward primer 5'-**CATATG**GCAAGTATTCCTAGCAGT-3' and the reverse primer 5'-**CTCGAG**TGTAGATACTTGGT-3' (restriction sites, shown in bold, were incorporated into the two primers). The PCR product was cloned into the pGEM-T Easy cloning vector (Promaga) and verified by DNA sequencing. The *sbd* gene fragment was subsequently ligated into pET23a(+) expression vector (Novagen) at *Nde*I and *Xho*I sites to generate pET-SBD. SBD-6H encoded by pET-SBD contains a C-terminal His$_6$ tag. The construct was transformed into competent *Escherichia coli* BL21-Gold (DE3) (Novagen) for protein expression.

(B) EXPRESSION AND PURIFICATION OF SBD-6H

BL21-Gold (DE3) cells transformed with pET-SBD were grown in LB medium containing 100 g/ml ampicillin at 37 °C until the $OD_{600}$ reached 0.6. The temperature was then reduced to 20 °C, and isopropyl -D-thiogalactoside (IPTG) was added to a final concentration of 400 M to induce recombinant protein expression. After further incubation for 16 h, cells were harvested by centrifugation at 3,700 g for 15 min at 4 °C, the resultant pellet was resuspended in 20 ml of binding buffer (50 mM sodium acetate, pH5.5) and then homogenized (EmulsiFlex-C5 homogenizer). The cell debris was removed by centrifugation at 16,000 g for 15 min at 4 °C. The supernatant was applied directly to the granular corn starch (pre-washed with binding buffer) and then incubated at 25 °C

for 30 min with gentle shaking. The starch was washed with 10 column volumes of binding buffer twice and then eluted with 2 column volumes of elution buffer (50 mM glycine/NaOH, pH10). The solution was dialyzed against binding buffer employing an Amicon stirred-cell concentrator (Millipore) equipped with a PM-10 membrane (10 kDa cut-off). Each fraction was analyzed by 15% SDS-PAGE gel and then stained with Coomassie brilliant blue. The protein concentrations were assayed by bicinchoninic acid (BCA) reagent kit (Pierce).

SBD-6H fused was expressed in *E. coli* BL21-Gold (DE3) at an optimized condition. After induction period, the soluble fraction was one-step purified by granular corn starch. Since low elution efficiency of SBD with maltose as elution agent employing previously reported methods was observed (Paldi et al. Biochem. J. (2003) 372: 905-910), here a substitute procedure for the elution of SBD with glycine/NaOH (pH10) was adopted. Purified SBD-6H was analyzed by 15% SDS-PAGE and stained with Coomassie brilliant blue as shown in Figure 1. SBD-6H derived from the signal step purification was homogeneous. This modified method enables fast and efficient purification of SBD from bacterial lysate with high purity (>98%) and yield (>90%).

EXAMPLE 2

EFFECT OF pH ON BINDING ABILITY AND STABILITY

For the measurement of binding ability, SBD-6H and granular corn starch were incubated in buffers with varied pH values for 1 h as described in the starch binding assay. The stability was measured by keeping SBD-6H at 25 °C for 30 min in different buffers including glycine/HCl (pH3), sodium acetate/acetic acid (pH4-5), $Na_2HPO_4/NaH_2PO_4$ (pH6-7), Tris/HCl (pH8) and glycine/NaOH (pH9-10). The remaining binding ability was measured at 25 °C for 1 h in sodium acetate buffer (50 mM, pH5.5). The relative binding ability of SBD-6H assayed at pH5 was normalized as 100%.

The stability of SBD-6H was analyzed in the pH range from 3 to 10 and it indicated that the SBD-6H was stable in a wide pH range, even at the extreme acidic and alkaline conditions (Figure 2). The binding assays were used to identify the optimal pH range of SBD-6H on adsorption of insoluble starch. As shown in Figure 2, the pH range for maximal SBD-6H binding was 5 to 6, whereas relative binding ability at pH4 and pH8 was only 60.6% and 55.1%, respectively.

As shown in Figure 2, SBD-6H was stable in a broad pH range, even at the extreme of the acidic and alkaline pH. It also indicated that the binding ability of SBD to starch was pH-dependence. The optimal pH for binding was around 5 to 6 similar to previous studies used maltose to release the SBD from starch even though maltose was not the optimal elution agent. Here, we had overcome this problem based on the properties of the pH-dependent binding ability. The elevated elution efficiency (>90%) was performed using an alkaline buffer as an elution agent.

EXAMPLE 3

ASSOCIATION RATE OF SBD-6H TO INSOLUBLE STARCH

The purified SBD-6H in the concentration range from 15.6 to 27.2 $\mu$M was added to 1 mg/ml of pre-washed granular corn starch and incubated at 25 °C with gentle stir for 5 h. The binding was terminated at different time intervals by sedimentation of the starch. After centrifugation at 16,000 g for 10 min at 4 °C, the protein concentration of the supernatant (unbound protein) was determined by the BCA assay, and the amount of bound protein was calculated from the difference between the initial and unbound protein concentrations. The bound protein at equilibrium expressed as micromole of protein per gram of starch was a linear function of the free (unbound) protein in the range of protein concentrations assayed.

The adsorption kinetics of SBD-6H to granular corn starch was performed at different protein concentrations as shown in Figure 3. About 50% of the SBD-6H was bound to the insoluble starch during the first 10 min (relative to the amount of bound protein at equilibrium state). In the initial period (up to 40 min), the adsorption exhibited a linear phase. The amount of bound proteins was in proportion to the incubation time.

Similar association rates (0.18 $\pm$ 0.1 $\mu$mol/min·g), indicated by the initial slope of the binding curves at different SBD-6H concentrations were observed. After the linear phase (after 40 min), the bound protein increased slowly within prolonged incubation time. At the time of 120 min, more than 95% relative binding was reached. The time required for reaching the equilibrium was similar to *Aspergillus* SBDs (Paldi et al. Biochem. J. (2003) 372: 905-910).

EXAMPLE 4

STARCH BINDING ASSAY

The starch-binding isotherm was analyzed as saturation binding assay: the SBD-6H was mixed with 1 mg/ml of pre-washed granular corn starch and incubated at 25 °C with gentle stir for 16 h. After centrifugation at 16,000 g for 10 min at 4 °C, the amount of bound protein was calculated. Controls with protein but no starch were included to ensure that no precipitation occurred during the period of assay. The dissociation constant ($K_d$) and the maximal amount of bound protein ($B_{max}$) were determined by fitting to the non-linear regression of the binding isotherms, and equation (1) was used for one binding site saturation binding.

$$B = B_{max} F/( K_d +F) \qquad (1)$$

where B ($\mu$mol) represents the bound protein; $B_{max}$ ($\mu$mol) was the maximal amount of bound protein; F ($\mu$mol) was the free protein in the system and $K_d$ ($\mu$mol) was the equilibrium dissociation constant. The units of the $B_{max}$ and $K_d$ values calculated were converted into $\mu$mol/g and $\mu$M, respectively.

SBD-BINDING AFFINITY AND CAPACITY

Starch-binding isotherm as shown in Figure 4 was used to study the affinity and capacity of purified SBD-6H. Equation (1) was used to calculate the non-linear regression curves of the equilibrium isotherm and determine the binding parameters ($B_{max}$ and $K_d$). The binding parameters, $K_d$ and $B_{max}$, determined by a two-parameter model for one binding site saturation binding, were $1.43 \pm 0.14$ $\mu$M and $41.14 \pm 1.05$ $\mu$mol/g, respectively. In addition, the dissociation constants from the engineered and proteolytically produced *Aspergillus* SBDs ($3.2 \pm 0.9$ $\mu$M and $12.7 \pm 0.5$ $\mu$M, respectively) were approximately 2-10 times higher than that of SBD-6H (Paldi et al. Biochem. J. (2003) 372: 905-910; Belshaw et al. FEBS Lett. (1990) 269: 350-353). These observations indicate that SBD-6H has better binding affinity to insoluble starch than *Aspergillus* SBD. On the other hand, the $B_{max}$ values of the engineered and proteolytically produced *Aspergillus* SBDs (0.56 $\mu$mol/g and $1.08 \pm 0.02$ $\mu$mol/g, respectively) were markedly different from SBD-6H. The $K_d$ of SBD-6H is approximately 2-fold smaller than that of *Aspergillus* SBDs, whereas the $B_{max}$ of SBD-6H is markedly about 70-fold higher than that of *Aspergillus* SBDs These results indicated that both the starch-binding affinity and capacity of SBD-6H are grater than *Aspergillus* SBDs.

EXAMPLE 5

(A) CONSTRUCTION OF SBD-eGFP

The eGFP gene fragment was subsequently ligated into pET-SBD at *Nco*I and *Xho*I sites to generate pSBD-eGFP. The plasmid construct was as shown in Figure 5. SBD-eGFP encoded by pSBD-eGFP contains a *N*-terminal SBD-6H and a *C*-terminal eGFP. The construct was transformed into competent *Escherichia coli* BL21-Gold (DE3) (Novagen) for protein expression.

(B) EXPRESSION AND PURIFICATION OF SBD-eGFP

BL21-Gold (DE3) cells transformed with pET-SBD were grown in LB medium containing 100 $\mu$g/ml ampicillin at 37 °C until the $OD_{600}$ reached 0.6. The temperature was then reduced to 20 °C, and isopropyl $\beta$-D-thiogalactoside (IPTG) was added to a final concentration of 400 $\mu$M to induce recombinant protein expression. After further incubation for 16 h, cells were harvested by centrifugation at 3,700 g for 15 min at 4 °C, the resultant pellet was resuspended in 20 ml of binding buffer (50 mM sodium acetate, pH5.5) and then homogenized (EmulsiFlex-C5 homogenizer). The cell debris was removed by centrifugation at 16,000 g for 15 min at 4 °C. The supernatant was applied directly to the granular corn starch (pre-washed with binding buffer) and then incubated at 25 °C for 30 min with gentle shaking. The starch was washed with 10 column volumes of binding buffer twice and then eluted with 2 column volumes of elution buffer (50 mM glycine/NaOH, pH10). The solution was dialyzed against binding buffer employing an Amicon stirred-cell concentrator (Millipore) equipped with a PM-10 membrane (10 kDa cut-off). Each fraction was analyzed by 15% SDS-PAGE gel and then stained with Coomassie brilliant blue. The protein concentrations were assayed by bicinchoninic acid (BCA) reagent kit (Pierce).

Purified SBD-eGFP was analyzed by 15% SDS-PAGE and stained with Coomassie brilliant blue as shown in Figure 6.

EXAMPLE 6

(A) Structure-based Multiple Sequence Alignment

Secondary structure prediction was performed at the Jpred server (URL, http://www.compbio.dundee.ac.uk/~www-jpred/Cuff, J. A., Clamp, M. E., Siddiqui, A. S., Finlay, M. and Barton, G. J. (1998) JPred: a consensus secondary structure prediction server. Bioinformatics 14, 892-893) and the Network Protein Sequence Analysis (NPSA)server (URL, http://npsa-pbil.ibcp.fr/ Combet, C., Blanchet, C., Geourjon, C. and Deleage, G. (2000) NPS@: network protein sequence analysis. Trends Biochem. Sci. 25, 147-150). The results of secondary structure prediction were used for multiple sequence alignment of the four starch-binding domains of GA from *R. oryzae, A. adeninivorans, M. circinelloides and A. niger* using the ClustalX program (Thompson, J. D., Gibson, T. J., Plewniak, F., Jeanmougin, F. and Higgins, D. G. (1997) The CLUSTAL_X windows interface: flexible strategies for multiple sequence alignment aided by quality analysis tools. Nucleic Acids Res. 25, 4876-4882) with the option of the secondary structure profile alignment.

(B) Progressive Secondary Structure Correlation Algorithm

In this study, we developed a PSSC algorithm, which was based on combinatorial informatics of predicting secondary structures and functionally important residues from related sequences of protein families. PSSC is comprised of three major steps: (*1*) obtaining multiple secondary structures using existing prediction systems to assign positions and boundaries of α-helices and β-strands, (*2*) transforming each of the predicted secondary structures into a new sequence representation by symbolizing the α-helices and β-strands as well as labeling selected aromatic residues, Y, W, and F, predefined as constrained residues, and (*3*) performing progressive correlation operations on one protein sequence with a resolved three-dimensional structure as the template. Among all predicted secondary structures, the one with the highest score represents the best candidate in which to identify the key residues corresponding to those in the template structure. Each annotated segment of α-helix and β-strand was simplified by a symbol "α" and "β". respectively, and the loop structures were substituted with specified constrained residues or a symbol "N" when no aromatic residue could be allocated. In the last step, the correlation operations were performed to provide the best alignment with respect to the location of secondary structures and constrained aromatic residues. Different weightings for matching scores were assigned to enhance the secondary structure relationship. A score of 2 was assigned to any matched symbol "α" and "β", a score of 1 was given to a matched "Y", "W", "F", and "N", and a score of -1 was assigned as a mismatch penalty. The predicted secondary structures with the highest scores were selected for identification of key residues through further correlation alignment.

(C) Model Construction

A molecular model of *Ro*GACBM21 was generated by the protein-modeling server Swiss Model (Schwede, T., Kopp, J., Guex, N. and Peitsch, M. C. (2003) SWISS-MODEL: An automated protein homology-modeling server. Nucleic Acids Res. 31, 3381-3385) using the NMR structure of *An*GACBM20 (PDB code 1AC0 (Sorimachi, K., et al., (1996) Solution structure of the granular starch binding domain of glucoamylase from Aspergillus niger by nuclear magnetic resonance spectroscopy. J. Mol. Biol. 259, 970-987)) as the template. Based on the modified sequence alignment, residues 1-13 were deleted from the *Ro*GACBM21 model structure, as there were no corresponding residues in *An*GACBM20. Energy minimization of the model was performed with the GROMOS96 implementation of the DeepView. Molecular graphics were performed using WebLab ViewerLite.

(D) Circular Dichroism

Circular Dichroism (CD) spectra were recorded on an Aviv circular dichroism spectrometer (model 202) equipped with a 450-W xenon arc lamp. Far-UV spectra from 200 to 260 nm were performed in a rectangular quartz cuvette with 0.1 cm path length at 25 and 96 °C at a scan speed of 4 nm sec$^{-1}$ and a bandwidth of 0.5 nm. Each spectrum was an average of three consecutive scans and was corrected by subtracting the buffer spectrum.

(E) Construction of the RoGACBM21 Model

Six CBM families, CBM20, CBM21, CBM25, CBM26, CBM34, and CBM41, contain SBDs, but currently only the structures of CBM20, CBM25, CBM26 and CBM34 have been elucidated. Because these three-dimensional structures share an immunoglobulin-like topology (CATH code 2.60.40) (Pearl, F. et. al., (2005) The CATH Domain Structure Database and related resources Gene3D and DHS provide comprehensive domain family information for genome analysis. Nucleic Acids Res. 33, D247-251), it is proposed that all SBDs of GA possess similar three-dimensional structures, even though they do not have high sequence identity.

Because the CBM21 sequence does not have a high level of identity with any previously solved CBM20 structure, a novel structure-based multiple sequence alignment strategy was used to compare proteins that share the similar topology but are dissimilar in sequence. Secondary structures of the CBM21 SBD sequences used in this study were initially predicted using the consensus secondary structure prediction servers, Jpred and NPSA. These servers construct a consensus result using a number of secondary structure prediction methods. The consensus of the secondary structures for all CBM21 SBDs except *Aa*GACBM21 revealed eight β-strands, consistent with the structural characteristics of CBM20, whereas the seventh β-strand in *Aa*GACBM21 was replaced by an α-helix. Figure 7A presents the results of the structure-based multiple sequence alignment for CBM families 20 and 21. Most aromatic residues and the secondary structural elements did not appear to be aligned, although the aromatic residues Tyr[16], Tyr[33], Trp[47], and Tyr[86], conserved in CBM21 members, were located in the vicinity of the key ligand-binding residues Tyr[527], Trp[543], Tyr[556], and Trp[590] in *An*GACBM20. In an attempt to identify additional potential ligand-binding residues in *Ro*GACBM21, the PSSC algorithm was used for structure-based multiple sequence alignments. It could be hypothesized that strongly correlated results could be obtained not only from relative α-helical and β-stranded structures, but also from key residues on loop regions that participate in ligand binding to maintain functionality similar to the template. As shown in Figure 7B, secondary structural elements were well-aligned in terms of relative positions and lengths, and eight aromatic residues in *Ro*GACBM21 were conserved with the corresponding residues in *An*GACBM20.

The aromatic residues likely to be involved in ligand binding were further investigated by structure-based sequence alignment (Figure 8A), which was generated by the combination of PSSC and ClustalX. The results revealed several potential ligand binding residues (Tyr[32], Trp[47], Tyr[58], Tyr[67], Tyr[83], Tyr[93], and Tyr[94]) in *Ro*GACBM21. The molecular model of *Ro*GACBM21 (Figure 8B left panel) was generated from the residues spanning 14-106, as aligned with *An*GACBM20. The root mean square deviation of the superimposed backbone atoms (356 atoms) was 1.38 A, indicating that the model fit the template structure very well. Judging from the *An*GACBM20 structure with two ligand binding sites (Figure 8B right panel), we hypothesized that the ligand binding residues in *Ro*GACBM21 could also be grouped into two sites, namely site I (Tyr[32], Tyr[58], and Tyr[67]) and site II (Trp[47], Tyr[83], Tyr[93], and Tyr[94]), with Tyr[58] and Tyr[94] located on the side chains oriented toward the inside of the *Ro*GACBM21 structure and not involved in ligand binding.

The *Ro*GACBM21 model includes a three-stranded β-sheet and a five-stranded β-sheet (Figure 8C left panel). These two β-sheets consist of anti-parallel β-strands with the topological organizations (1↓2↑5↓) and (4↑3↓6↑7↓8↓). β-strands 2-8 of *Ro*GACBM21 could be superimposed onto β-strands 1-7 of *An*GACBM20 (Figure 8C right panel), and the first strand of *Ro*GACBM21 could possibly be superimposed onto the eighth β-strand of *An*GACBM20, although in the reverse direction.

(F) **All-β-strand Secondary Structure**

The CD spectrum of the native state *Ro*GACBM21 (Figure 9) had a trough at 215 nm, typical for a protein containing only β-strands, suggesting that *Ro*GACBM21 does not contain helical structure and that both β-strand and random coil structures significantly contribute to the overall conformation. This observation agrees with the results of the secondary structure prediction and the common all-β-strand structure of the starch-binding CBMs. The spectrum also contained a peak near 230 nm, an unusual feature that has been observed in some carbohydrate-binding domains in which disulfide bonds and aromatic side chains of phenylalanine, tyrosine and tryptophan may contribute to spectral peaks having positive ellipticity (Roberge, M., Lewis, R. N., Shareck, F., Morosoli, R., Kluepfel, D., Dupont, C. and McElhaney, R. N. (2003) Differential scanning calorimetric, circular dichroism, and Fourier transform infrared spectroscopic characterization of the thermal unfolding of xylanase A from Streptomyces lividans. Proteins 50, 341-354). *Ro*GACBM21 contains eighteen aromatic residues and no cysteine (Figure 8A), suggesting that the peak of positive ellipticity near 230 nm may be a consequence of aromatic residues.

EXAMPLE 7

(A) **Site-directed Mutagenesis**

All mutants of *Ro*GACBM21 were generated using the PCR-based QuikChange site-directed mutagenesis method (Stratagene) with pET-*Ro*GACBM21 as the template, two complementary primers containing the desired mutation and *Pfu* Turbo DNA polymerase (Stratagene). The sequence of each mutant plasmid was verified to confirm that additional PCR-induced mutations had not been introduced. All constructs were transformed into competent *E. coli* BL21-Gold (DE3) for protein expression.

### (B) UV Difference Spectra of Wild Type and Mutant RoGACBM21

All spectra were obtained using a UV/visible spectrophotometer (Hitachi U-3310) at 270 to 300 nm with a bandwidth of 0.5 nm. Spectra of 30 $\mu$M wild type or mutant *Ro*GACBM21 were recorded in 50 mM sodium acetate, pH 5.5, at 25 °C in the absence or presence of 500 $\mu$M $\beta$-cyclodextrin ($\beta$CD) (Sigma). Each difference spectrum, calculated by subtracting the protein-only spectrum from protein-ligand spectrum, was used to compare with difference spectra of model compounds *N*-acetyltryptophan (50 $\mu$M) (Sigma) and *N*-acetyltyrosine (100 $\mu$M) (Sigma), as perturbed in 20% DMSO.

### (C) Chemical Modification of Tryptophan Residues with N-bromosuccinimide (NBS)

Oxidation of tryptophan residues was performed by titrating native wild type and W47A *Ro*GACBM21 protein (30 $\mu$M, in 50 mM sodium acetate, pH 5.5), native wild type protein in the presence of 500 $\mu$M $\beta$CD, or of denaturant (8 M urea) with NBS (1 mM, freshly prepared) (Sigma) in a rectangular quartz cuvette with 1 cm path length at 25 °C. At each titration point, the mixture was incubated for 3 min, and the absorbance at 280 nm was recorded using a UV/visible spectrophotometer. The titration process was continued until the decreasing trend of absorbance at 280 nm stagnated or started to rise. The number of tryptophan residues oxidized by NBS was calculated using the absorbance method (Spande, T. F. and Witkop, B. (1967) Determination of the tryptophan content of proteins with N-bromosuccinimide. Methods Enzymol. 11, 498-506).

### (D) Chemical Modification of Tyrosine Residues with Tetranitromethane (TNM)

Nitration of tyrosine residues was performed using TNM as described by Sokolovsky et al., (1966) Tetranitromethane. A reagent for the nitration of tyrosyl residues in proteins. Biochemistry 5, 3582-3589. *Ro*GACBM21 (10 $\mu$M, in 100 mM Tris-HCl, pH8.0) was added into TNM (1 mM, in 95% ethanol) at 25 °C for 180 min, and the absorbance at 428 nm was monitored using a UV/visible spectrophotometer. The number of tyrosine residues oxidized by TNM was calculated using the molar extinction coefficient value of 4,100 $M^{-1}$ $cm^{-1}$ for 3-nitrotyrosine.

### (E) Distinct Roles of the Two Tryptophan Residues in RoGACBM21

NBS was employed to further evaluate the number of tryptophan residues exposed on the surface of *Ro*GACBM21. NBS modification of the tryptophan residues in *Ro*GACBM21 was monitored by recording the decrease in absorbance at 280 nm (Figure 10A). The decrease in $A_{280}$ of native *Ro*GACBM21 began to rise at the NBS:protein ratio of 2.5. This NBS-induced rise in absorbance at 280 nm is a phenomenon typically observed following the oxidation of tyrosine residues (Bray, M. R., Carriere, A. D. and Clarke, A. J. (1994) Quantitation of tryptophan and tyrosine residues in proteins by fourth-derivative spectroscopy. Anal. Biochem. 221, 278-284). However, no increase in the $A_{280}$ of denatured *Ro*GACBM21 was observed since none of the tyrosine residues in urea solution are able to be modified by NBS (Sokolovsky *et al.,* (1966)). As shown in Figure 10A, the number of modified tryptophan residues in native and denatured *Ro*GACBM21 was calculated to be 1.6 and 2.0, respectively. The only two tryptophan residues residing in *Ro*GACBM21 could be modified under native and denatured conditions, indicating that both Trp[47] and Trp[70] may be exposed on the surface of the protein.

To determine if two Trp residues are located within the ligand binding site(s), NBS oxidations were carried out in the presence of the ligand $\beta$CD. Pre-incubation of native *Ro*GACBM21 with $\beta$CD resulted in the apparent protection of one Trp residue from oxidation (Figure 10A), suggesting that only one tryptophan residue is involved in $\beta$CD binding.

To further probe which tryptophan residue are located in the ligand binding site, both Trp[47] and Trp[70] were individually mutated to alanine. The W47A mutant could be expressed in and purified from *E. coli;* however, the solubility of the W70A mutant was very poor under all expression conditions tested, suggesting that Trp[70] is quite important for the correct folding of *Ro*GACBM21. The native W47A mutant was subjected to treatment with NBS, and the result was consistent with that of the wild type *Ro*GACBM21 tested in the presence of $\beta$CD (Figure 10A), strongly suggesting that Trp[47] is the key ligand binding residue. This result was also confirmed by UV difference spectra for wild type and mutant *Ro*GACBM21 (Figure 10B), which were perturbed with 500 $\mu$M $\beta$CD and then compared with the model compounds *N*-acetyltryptophan and *N*-acetyltyrosine perturbed with 20% DMSO. The spectrum of wild type *Ro*GACBM21 (Figure 10B line c), with three peaks near 277, 285, and 293 nm, resembled the tryptophan and tyrosine perturbation spectra (Figure 10B lines a and b), suggesting that the perturbed tryptophan and tyrosine residue(s) in *Ro*GACBM21 are present in the ligand binding site(s). The difference spectrum of W47A (Figure 10B line e) was diminished and virtually identical to the baseline scan, revealing that residue Trp[47] is involved in soluble ligand binding. These data suggest that Trp[47] plays a critical role in binding to soluble ligands and that Trp[70] is essential for maintaining the structure.

## (F) Identification of Key Tyrosine Residues for ligand binding

The tyrosine residues in *Ro*GACBM21 were nitrated with TNM and the numbers of tyrosine residues exposed on the surface were calculated using the molar extinction coefficient of the nitrated tyrosine (3-nitrotyrosine) (Figure 10C). More than six of the twelve overall tyrosine residues were nitrated by TNM, suggesting that at least 50% of the tyrosines were exposed on the surface and thus may contribute to a potential ligand binding site(s). To further investigate the crucial tyrosine residues located in *Ro*GACBM21 were individually substituted with alanine.

Qualitative binding assays were carried out to probe the number and location of potential ligand binding residues in wild type *Ro*GACBM21, the tryptophan mutant (W47A) and tyrosine mutants (Y32A, Y58A, Y67A, Y83A, Y93A, and Y94A). As shown in Figure 11, substitution at Tyr[32], Trp[47], Tyr[67], Tyr[83], and Tyr[93] with alanine significantly reduced the binding to insoluble starch; as expected, no change was observed in mutants Y58A and Y94A. These results validated our assumptions based simply on the three-dimensional model of *Ro*GACBM21. Nevertheless, the solubility of Y67A, Y83A, and Y93A was much lower than the other mutants, indicating that Tyr[67], Tyr[83], and Tyr[93] are also important for stabilizing the *Ro*GACBM21 structure. Accordingly, no soluble double-mutant protein involving these residues was obtained. Therefore, subsequent studies focused on Tyr[32] and Trp[47].

The difference UV spectra of mutant Y32A (Figure 10B line d) and wild type *Ro*GACBM21 did not differ significantly, indicating that binding site I is not involved in βCD binding. It should be noted that putative binding residues Tyr[16] and Tyr[86] inferred from direct sequence alignment are not involved in the binding to either soluble (Figure 10 lines f and g) or insoluble ligand (Figure 11).

EXAMPLE 8

### (A) Qualitative Measurement of Binding to Insoluble Starch

The wild type and mutant *Ro*GACBM21 (16 $\mu$M, 100 $\mu$l) in 50 mM sodium acetate, pH5.5, were each mixed with 0.1 mg of insoluble starch (Sigma), and the mixture was incubated at 25 °C with gentle stirring for 5 h, after which the insoluble starch was pelleted by centrifugation at *13,000 g* for 2 min at 4 °C. The pellet (the bound fraction) and supernatant (the unbound fraction) were analyzed by SDS-PAGE using a 15% gel. To ensure that no precipitation occurred during the assay period, protein with no insoluble starch was included as a control.

### (B) Quantitative Measurement of Binding to Insoluble Starch

The starch-binding isotherm was analyzed as a saturation binding assay. Wild type and mutant *Ro*GACBM21 (100 $\mu$l, in 50 mM sodium acetate, pH5.5) were each mixed with 0.1 mg of pre-washed insoluble starch and incubated at 25 °C with gentle stirring for 16 h. After centrifugation at 16,000 *g* for 10 min at 4 °C, the protein concentration of the supernatant (unbound protein) was determined using the BCA assay, and the amount of bound protein was calculated from the difference between the initial and unbound protein concentrations. The dissociation constant ($K_d$) and the maximal amount of bound protein ($B_{max}$) were determined by fitting to the nonlinear regression of the binding isotherms using a standard single-site binding model.

### (C) Quantitative Measurement of Binding to Soluble Polysaccharides

Fluorescence spectrophotometry of the binding of wild type and mutant *Ro*GACBM21 to soluble polysaccharides (Sigma) was recorded by measuring changes in the intrinsic protein fluorescence intensity. Experiments were performed in 50 mM sodium acetate, pH5.5, at 25 °C using a Perkin-Elmer LS-55 spectrophotometer. Circular and linear carbohydrates (2-20 mM) were titrated into *Ro*GACBM21 (10 $\mu$M, 2 ml), and the fluorescence-emission spectrum was monitored at 350 nm with a fixed excitation at 280 nm. The relative changes in fluorescence intensity were plotted against ligand concentration, and the data were fitted to a simulated curve using the appropriate equation for a single binding site.

### (D) Data analysis

Data analyses of kinetic and affinity parameters were performed using GraphPad Prism (GraphPad Software).

(E) **Two Binding Sites with Distinct Ligand Specificities**

Equilibrium starch-binding isotherm data for purified *Ro*GACBM21 were fitted to non-linear regression curves (Figure 12). The binding parameters, $K_d$ and $B_{max}$, for *Ro*GACBM21, as determined by a two-parameter model for single-site saturation binding, were 1.43 $\pm$ 0.14 $\mu$M and 41.14 $\pm$ 1.05 $\mu$mol g$^{-1}$, respectively (Table I). However, the dissociation constants for the engineered *An*GACBM20 (3.2 $\pm$ 0.9 $\mu$M) and proteolytically produced *An*GACBM20 (12.7 $\pm$ 0.5 $\mu$M) (Paldi, T., et al., (2003) Glucoamylase starch-binding domain of Aspergillus niger B1: molecular cloning and functional characterization. Biochem. J. 372, 905-910 and Belshaw, N. J. et. al. (1990) Production and purification of a granular-starch-binding domain of glucoamylase 1 from Aspergillus niger. FEBS Lett. 269, 350-353) were approximately 2- and 10-fold higher, respectively. Furthermore, the $B_{max}$ values for the engineered *An*GACBM20 (0.56 $\mu$mol/g) and proteo-lytically produced *An*GACBM20 (1.08 $\pm$ 0.02 $\mu$mol g$^{-1}$) were 70- and 40-fold lower, respectively than that of *Ro*GACBM21. It should be noted though that the ligand binding capacity may be affected by the varying ratios of amylopectin to amylose, the size and shape of starch granules, or the swelling affect derived from how long the starch sits in the buffer.

Table I. Affinity of RoGACBM21 derivatives for insoluble starch determined by depletion isotherms

| Protein | $K_d$ ($\mu$M) | $B_{max}$ ($\mu$mol/g) |
|---|---|---|
| WT | 1.4 $\pm$ 0.1 | 41.1 $\pm$ 1.1 |
| Y32A | 0.8 $\pm$ 0.1 | 23.4 $\pm$ 0.7 |
| W47A | 4.4 $\pm$ 0.6 | 23.0 $\pm$ 1.3 |
| Y32A/W47A | 25.0 $\pm$ 2.9 | 5.1 $\pm$ 0.3 |

The quantitative binding isotherm and Scatchard analysis (Figure 12 and Table I) revealed that the binding capacity of insoluble starch for both mutants was reduced to approximately 50 % relative to that of the wild type. The binding affinity of the Y32A and W47A mutants were similar to that of the wild type, and the binding of the Y32A/W47A double mutant to insoluble starch was almost completly abolished. The data for quantitative binding to soluble ligands is presented in Table II. For cyclic carbohydrates, the affinity of wild type *Ro*GACBM21 for $\beta$CD ($K_d$ = 5.1 $\pm$ 0.7 $\mu$M) was slightly stronger than for $\gamma$CD ($K_d$ = 8.3 $\pm$ 1.5 $\mu$M), and the binding to $\alpha$CD ($K_d$ > 300 $\mu$M) was relatively poor. For linear carbohydrates, the $K_d$ values of wild type *Ro*GACBM21 decreased with increasing ligand length. These results indicate that the distance and the orientation between the aromatic side chains residing in the binding site are the major determinants for ligand binding to *Ro*GACBM21. Therefore, the binding to cyclic carbohydrates was dependent on the radian of the ligand surface, whereas binding to linear carbohydrates was governed by ligand length. For the ligand with $\alpha$-1,6-glycosidic linkage, the affinity of *Ro*GACBM21 for isomaltotriose ($K_d$ = 3.8 $\pm$ 0.7 $\mu$M) was stronger than those of the soluble ligands with $\alpha$-1,4-glycosidic linkage, suggesting that *Ro*GACBM21 may prefer to bind to the branch of the starch.

Affinities of the W47A mutant for the soluble carbohydrates used in this study were either too low to determine by fluorescence titration spectra or substantially affected by the alanine substitution. The results indicate that Trp[47] is indeed the major residue involved in binding to both soluble and insoluble ligands. The Y32A mutant and wild type *Ro*GACBM21 showed similar affinities for various cyclodextrins, confirming that Tyr[32] is not involved in binding to cyclodextrin. In addition, the effect of the length of linear oligosaccharides on the binding to Y32A was relatively insignificant compared with that of wild type *Ro*GACBM21, suggesting that binding site I may facilitate the binding of *Ro*GACBM21 to longer linear oligosaccharides.

Table II. Affinity of RoGACBM21 derivative for soluble oligosaccharides determined by Fluorescence titration spectra

| Ligands | $K_d$ ($\mu$M) | | |
|---|---|---|---|
| | WT | Y32A | W47A |
| $\gamma$CD | 8.3 $\pm$ 1.5 | 6.6 $\pm$ 0.8 | NB |
| $\beta$CD | 5.1 $\pm$ 0.7 | 6.0 $\pm$ 0.7 | NB |
| $\alpha$CD | 333.0 $\pm$ 13.1 | 286.3 $\pm$ 30.5 | NB |
| Maltoheptaose | 5.5 $\pm$ 1.0 | 44.7 $\pm$ 5.3 | NB |
| Maltohexaose | 24.4 $\pm$ 1.7 | 46.4 $\pm$ 5.8 | NB |
| Maltopentaose | 27.5 $\pm$ 1.4 | 50.2 $\pm$ 6.2 | NB |
| Maltotetraose | 54.4 $\pm$ 4.8 | 53.3 $\pm$ 5.5 | NB |
| Maltotriose | 61.9 $\pm$ 6.0 | 58.2 $\pm$ 8.1 | NB |

(continued)

| Ligands | $K_d$ (μM) | | |
|---------|-----|------|------|
|  | WT | Y32A | W47A |
| Isomaltotriose | 3.8 ± 0.7 | 3.9 ± 0.8 | NB |

NB, no binding detected.

Unlike the starch-binding CBM20s having two binding sites with similar binding affinities (Williamson, M. P. et al., (1997) Function of conserved tryptophans in the Aspergillus niger glucoamylase 1 starch binding domain. Biochemistry 36, 7535-7539 and Giardina, T. et al., (2001) Both binding sites of the starch-binding domain of Aspergillus niger glucoamylase are essential for inducing a conformational change in amylose. J. Mol. Biol. 313, 1149-1159), RoGACBM21 contains two binding sites with distinct binding specificities, indicating that each plays a different but cooperative role in carbohydrate-protein recognition. The binding site II is the major binding site for soluble and insoluble ligands, and the binding site I is involved in assisting RoGACBM21 in association with long chain carbohydrates.

EXAMPLE 9

The method of the present invention could be applied to predict the enzymes and proteins containing the CBM20 and CBM21 modules. All sequences were retrieved from GenBank except for the cgtBacma2 (UniProt: P 31835). The predicted residues Y, W and F of ligand binding site of these enzymes and proteins were underlined as follows:

amYAspka/538-634    TLPITFEELV TTTYGEEVYL SGSISQLGEW HTSDAVKLSA DDYTSSNPEW SVTVSLPVGT

TFEYKFIKVD EGGSVTWESD PNREYTVPEC GSGSGET

amYAspnd/521-617    AITVVFQERV QTAYGENVFL AGSISQLGNW DTTEAVALSA AQYTATDPLW TVAIELPVGT

SFEFKFLKKR QDGSIVWESN PNRSAKVNEG CARTTQT

amYBacsp/515-608    TSNVTFTVNN ATTTSGQNVY VVANIPELGN WNTANAIKMN PSSYPTWKAT IALPQGKAIE

FKFIKKDQAG NVIWESTSNR TYTVPFSSTG SYTA

amYCrYsp/528-624    AGTVVFDVYV QTQYGQSVVI AGNIPQLGNW SPANGLNLNA NQYTASSPKW

TGTITGVAPG TTFQWKPIVV TNGNDNWYPG NNQQATTGSA CSSPAAD

amYStrgr/470-562    QTSASFHVNA TTAWGENIYV TGDQAALGNW DPARALKLDP AAYPVWKLDV

PLAAGTPFQY KYLRKDAAGK AVWESGANRT ATVGTTGALT LND

amYStrlm/470-562    QTSASFHVNA TTAWGENIYV TGDQAALGNW DPARALKLDP AAYPVWKLDV

PLAAGTPFQY KYLRKDAAGK AVWESGANRT ATVGTTGALT LND

amYStrli1/480-570    RRVPSAVDAT TSWGQNIYVT GNRPELGNWN PGGALQLDPA AYPVWKRDVE

LPEGTTFEYK YLRKDDAGNV TWESGANRTA TVNTTKTTLN D

amYStrli2/478-569    SSGVSFAVDA TTSWGQDIYV TGNRPGLGHW DPGGGLQLDP AAYPVWKRDV

ELPEGTTFEY KSLRKADAGN VTWESGANRT ATVNTTKTTL ND

amYStrvi/473-565    TSGASFNVTA TTVVGQNIYV TGNRAELGNW APASALKLDP ATYPVWKLTV GLPAGTSFEY

KYIRKDAAGN VTWESGANRT ATVPASGQLV LND

amYThncu/505-597    TVTARFHATV TTWYGQEVAV VGSIPELGSW QPAQGVRLRT DSGTYPVWSG

AVDLPAGVGF EYKYVKLNRT APWSGSRAAT ASPPWMTSGG GCS

amYAspaW/534-630    ) TLPITLEELV TTTYGEEIYL SGSISQLGEW DTSDAVKLSA DDYTSSNPEW YVTVSLPVGT

TFEYKFIKVE EDGSVTWESD PNREYTVPEC GSGETVV

atrActsp/622-720    PVPVQFTVQN PPATAPGESL YLTGDVAELG HWSTSPDQTA GQLLRVPNES RGVLVADLPA

GAPVEFKFVK VAADGTVTWE GGANHRYTVP AGGTGTTSL

**15**

(continued)

cgtBacag/580-677

QVTVRFIIDN AETKLGENVF LVGNVHELGN WDPEQSVGRF FNQIVYQYPT WYYDVNVPAN TDLEFKFIKI DQDNNVTWQS GANQTYSSPE SGTGIIRV

cgtBacbr/592-692

QVSVRFAVNN ATTNSGTNVY IVGNVSELGN WDPNKAIGPM FNQVMYKYPT WYYDISVPAG KNLEYKYIKK DHNGNVTWQS GNNRTYTSPA TGTDTVISNW

cgtBacci2/613-709

QVSVRFVVNN ATTALGQNVY LTGSVSELGN WDPAKAIGPM YNQVVYQYPN WYYDVSVPAG KTIEFKFLKK QGSTVTWEGG SNHTFTAPSS GTATINV

cgtBacci8/613-709

QVSVRFVVNN ATTALGQNLY LTGNVSELGN WDPAKAIGPM YNQVVYQYPN WYYDVSVPAG KTIEFKFLKK QGSTVTWEGG SNHTFTAPSS GTATINV

cgtBacciA/613-709

QVSVRFVVNN ATTALGQNVY LAGSVSELGN WDPAKAIGPL YNQVIYQYPT WYYDVTVPAG KTIEFKFLKK QGSTVTWEGG SNHTFTAPTS GTATINV

cgtBaccl/601-698

QESVRFVVDN AHTNYGENVY LVGNVPELGN WNPADAIGPM FNQVVYSYPT WYYDVSVPAD TALEFKFIIV DGNGNVTWES GGNHNYRVTS GSTDTVRV

cgtBacli/618-715

QVSVRFVINN ATTALGENIY LTGNVSELGN WTTGAASIGP AFNQVIHAYP TWYYDVSVPA GKQLEFKFFK KNGATITWEG GSNHTFTTPT SGTATVTI

cgtBacma1/614-710

QVTVRFLVNQ ANTNYGTNVY LVGNAAELGT WDPNKAIGPM YNQVIAKYPS WYYDVSVPAG TKLDFKFIKK GGGTVTWEGG GNHTYTTPAS GVGTVTVDWQ N

cgtBacma2/613-713

QVTVRFKVNN ATTALGQNVY LTGNVAELGN WTAANAIGPM YNQVEASYPT WYFDVSVPAN TALQFKFIKV NGSTVTWEGG NNHTFTSPSS GVATVTVDWQ N

cgtBacoh/604-701

QVSIRFAVNN ATTSLGTNLY MVGNVNELGN WDPDQAIGPM FNQVMYQYPT WYYDISVPAE ENLEYKFIKK DSSGNVVWES GNNHTYTTPA TGTDTVLV

cgtBacsp0/613-709

QVTVRFVINN ATTALGQNVF LTGNVSELGN WDPNNAIGPM YNQVVYQYPT WYYDVSVPAG QTIEFKFLKK QGSTVTWEGG ANRTFTTPTS GTATVNV

cgtBacsp1/604-701

QVSVRFGVNN ATTSPGTNLY IVGNVNELGN WDADKAIGPM FNQVMYQYPT WYYDISVPAG KNLEYKYIKK DQNGNVVWQS GNNRTYTSPT TGTDTVMI

cgtBacsp7/613-709

QVSVRFVVNN ATTALGQNVY LAGSVSELGN WDPAKAIGPL YNQVIYQYPT WYYDVTVPAG KTIEFKFLKK QGSTVTWEGG SNHTFTAPTS GTATINV

cgtBacsp3/612-708

QVTVRFVINN ATTALGQNVF LTGNVSELGN WDPNNAIGPM YNQVVYQYPT WYYDVSVPAG QTIEFKFLKK QGSTVTWEGG ANRTFTTPTS GTATVNV

cgtBacsp63/618-715

QVTVRFVINN ASTTLGQNIY LTGNVAELGN WSTGSTAIGP AFNQVIHQYP TWYYDVSVPA GKELEFKFFK KNGSTITWEG GSNHKFTTPA SGTATVTV

cgtBacsp6/604-701

QVSIRFAVNN ATTSLGTNLY IVGNVNELGN WDPDQAIGPM FNQVMYQYPT WYYDISVPAE ENLEYKFIKK DSSGNVVWES GNNHTYTTPA TGTDTVLV

cgtBacspB/613-709

QVSVRFVVNN ATTALGQNLY LTGNVSELGN WDPAKAIGPM YNQVVYQYPN WYYDVSVPAG KTIEFKFLKK QGSTVTWEGG SNHTFTAPSS GTATINV

cgtBacspD/598-699

QTSVRFVVNQ AETSVGENLY VVGDVPELGS WDPDKAIGPM FNQVLYSYPT WYYDVSVPAN QDIEYKYIMK DQNGNVSWES GNNHIYRTPE NSTGIVEVNF NQ

cgtBacspK/626-723

QVSVRFGVNN ATTSPGTNLY IVGNVNELGN WDADKAIGPM FNQVMYQYPT WYYDISVPAG KNLEYKYIKK DQNGNVVWQS GNNRTYTSPT TGTDTVMI

(continued)

| | |
|---|---|
| cgtBacst/610-711 | QVSVRFVVNN ATTNLGQNIY IVGNVYELGN WDTSKAIGPM FNQVVYSYPT WYIDVSVPEG KTIEFKFIKK DSQGNVTWES GSNHVYTTPT NTTGKIIVDW QN |
| cgtGeost/610-707 | QVSVRFVVNN ANTNWGENIY LVGNVHELGN WNTSKAIGPL FNQVIYSYPT WYVDVSVPEG KTIEFKFIKK DGSGNVIWES GSNHVYTTPT STTGTVNV |
| cgtKlepn/559-654 | VQSINFTCNN GYTISGQSVY IIGNIPQLGG WDLTKAVKIS PTQYPQWSAS LELPSDLNVE WKCVKRNETN PTANVEWQSG ANNQFNSNDT QTTNGS |
| cgtThetu/610-706 | QICVRFVVNN ASTVYGENVY LTGNVAELGN WDTSKAIGPM FNQVVYQYPT WYYDVSVPAG TTIQFKFIKK NGNTITWEGG SNHTYTVPSS STGTVIV |
| cgtThcsp/634-735 | QIPAIFEVRN TQGTNLETQV GEFLWLTGSV PELSYWSPET IKAVGPMLCP GWPDWFVVAS VPADTYIEFK FLKAPLGGTG IWEVGSNHAY LTPSSGIGEV SV |
| cgt_Bacsp5/612-712 | QVTVRFVINN ATTALGQNVF LTGNVSELGN WDPNNAIGPM YNQVVYQYPT WYYDVSVPAG QTIEFKFLKK QGSTVTWEGG ANRTFTTPTS GTATVNVNWQ P |
| cgt_Glovi/542-637 | REIVRIQVNG FRTQPGEVVA VIGDCPELGD WDLSRAFRLE YINDNTWFGE IPFNKSANQI VAYKYVIFRE NGPPINENRT SRRRFVPDKS IAKWRD |
| cgt_Nossp7/539-634 | QTIVRVQLNG VHTQPGETIV VVGDCPELGN WDISKAYPLE YINSNTWFAE IPFDESAGKL ISYKYAMWRE GRSPLRENIL NRRWVVAKEG TVKWRD |
| cgt_Nossp9/539-634 | QTIVRAQLNG VQTQPGETIV VIGDCPELGN WDISKAYPLE YINTNTWFAE IPFNESAGKL IAYKYALWRE GQSPLRENLV NRRWVIAKEG TVKWRD |
| cgt_StcpY/611-710 | QIPVRLLIND FKTVPGEQLY LMGDVFEMGA NDAKNAVGPL FNNTQTIAKY PNWFFDTHLP INKEIAVKLV KKDSIGNVLW TSPETYSIKT GHEAQTITIK |
| m5hPsespK/514-609 | VLSLTFNETA DTVWGQNLFV VGNVGALGNW APAAGAAMTW ISGSGSTGQW RATVQLPADT PVQYKYVKKD GAGNVVWESG GNRVVTTPAP GATIAV |
| m4hPsesa/454-550 | LVNVNFRCDN GVTQMGDSVY AVGNVSQLGN WSPASAVRLT DTSSYPTWKG SIALPDGQNV EWKCLIRNEA DATLVRQWQS GGNNQVQAAA GASTSGS |
| m4hPsest/451-547 | LVSVSFRCDN GATQMGDSVY AVGNVSQLGN WSPAAALRLT DTSGYPTWKG SIALPAGQNE EWKCLIRNEA NATQVRQWQG GANNSLTPSE GATTVGR |
| maaBacst/614-715 | QTSVVFTVKS APPTNLGDKI YLTGNIPELG NWSTDTSGAV NNAQGPLLAP NYPDWFYVFS VPAGKTIQFK FFIKRADGTI QWENGSNHVA TTPTGATGNI TV |
| apuBacst/1339-1458 | MVQVTFKVRA PSYTPLDTRI TIPNSLNGWN TGAWEMTRGG AVTSDWEFTT ELQEGETIIY KYVKGASWDQ EGLADHTRDD QTDDDVSYYG YGAIGTDLKI TVQNQGNNKM IIQDYILRWI |
| apuBacspX/1335-1454 | PDIVMVQVTF KVKAPSYTPL DTRITIPNSI NGWNTGAWEM TRGGAVTPDW EFTTELQEGE TITYKVKGG SWDQEGLADH TRDDQTDDDV SYYGYGAIGT ELKVTVHNQG NNKMVIQDYI LRWI |
| apuTheth/1251-1353 | PIKVIFNVTV PDYTPDAVNL AGTFPNATWD PSAQQMTKID NNTYSITLTL DEGTQIEYKY ARGSWDKVEK DEYGNEFASN RKVTIVNQGN NEMTINDTVY RWR |
| apuTheet/1254-1356 | PIKVIFNVTV PDYTPDDGAN IAGNFHDAFW NPSAHQMTKT GPNTYSITLT LNEGTQLEYK YARGSWDKVE KGEYGEEIAN RKITVVNQGS NTMVVNDTVQ RWR |

(continued)

| | |
|---|---|
| apuThetc/1254-1361 | PIKVTFNVTI PDYTPDDGVN IAGNFPDAFW NPNANQMTKA GSNTYSITLT LNEGTQIEYK YARGSWDKVE KGEYGNEIDN RKITVVNQGS NTMVVNDTVQ RWR |
| bmYBacce/452-545 | QTIVVKNVPT TIGDTVYITG NRAELGSWDT KQYPIQLYYD SHSNDWRGNV VLPAERNIEF KAFIKSKDGT VKSWQTIQQS WNPVPLKTTS HTSS |
| bmYBacme/452-544 | QTVVVKNAPT ALGETVYIVG DRAELGQWDT SIYPIKLTYN SSTADWRGTV HFPASQNVQF KAIVKRADGS LKAWQPSQQY WSVPSTTTTY TDN |
| bmYCloth/453-547 | TIPVTFTINN ATTYYGQNVY IVGSTSDLGN WNTTYARGPA SCPNYPTWTI TLNLLPGEQI QFKAVKIDSS GNVTWEGGSN HTYTVPTSGT GSVTI |
| gmYAspaW/537-633 | AVAVTFDLTA TTTYGENIYL VGSISQLGDW ETSDGIALSA DKYTSSNPLW YVTVTLPAGE SFEYKFIRVE SDDSVEWESD PNREYTVPQA CGESTAT |
| gmYAspFi/538-634 | AVAVTFDLTA TTTYGENIYL VGSISQLGDW ETSDGIALSA DKYTSSDPLW YVTVTLPAGE SFEYKFIRIE SDDSVEWESD PNREYTVPQA CGTSTAT |
| gmYAspka/537-633 | AVAVTFDLTA TTTYGENIYL VGSISQLGDW ETSDGIALSA DKYTSSNPLW YVTVTLPAGE SFEYKFIRVE SDDSVEWESD PNREYTVPQA CGESTAT |
| gmYAspni/538-634 | AVAVTFDLTA TTTYGENIYL VGSISQLGDW ETSDGIALSA DKYTSGDPLW YVTVTLPAGE SFEYKFIRIE SDDSVEWESD PNREYTVPQA CGTSTAT |
| gmYAspor/511-606 | TVSVTFAVKA TTVYGESIKI VGSISQLGSW NPSSATALNA DSYTTDNPLW TGTINLPAGQ SFEYKFIRVQ NGAVTWESDP NRKYTVPSTC GVKSAV |
| gmYAspsh/537-633 | AVAVTFDLTA TTTYGENIYL VGSISQLGDW ETSDGIALSA DKYTSSNPPW YVTVTLPAGE SFEYKFIRVE SDDSVEWESD PNREYTVPQA CGESTAT |
| gmYCorro/482-579 | SVEVTFDVYA TTVYGQNIYI TGDVSELGNW TPANGVALSS ANYPTWSATI ALPADTTIQY KYVNIDGSTV IWEDAISNRE ITTPASGTYT EKDTWDES |
| gmYHorre/506-602 | QVSITFNINA TTYYGENLYV IGNSSDLGAW NIADAYPLSA SAYTQDRPLW SAAIPLNAGE VISYQYVRQE DCDQPYIYET VNRTLTVPAC GGAAVTT |
| gmYHumgr/514-611 | EVYVTFNERV STAWGETIKV VGNVPALGNW DTSKAVTLSA SGYKSNDPLW SITVPIKATG SAVQYKYIKV GTNGKITWES DPNRSITLQT ASSAGKCA |
| gmYLened/476-568 | AVSVTFNVDA STLEGQNVYL TGAVDALEDW STDNAILLSS ANYPTWSVTV DLPGSTDVQY KYIKKDGSGT VTWESDPNME ITTPANGTYA TND |
| gmYNeucr/525-621 | EVLVTFNEKV TTSYGQTVKV VGSIAALGNW APASGVTLSA KQYSSSNPLW STTIALPQGT SFKYKYVVVN SDGSVKWEND PDRSYAVGTD CASTATL |
| gmYTalem/516-612 | SVAVTFDEIV STSYGETIYL AGSIPELGNW STASAIPLRA DAYTNSNPLW YVTVNLPPGT SFEYKFFKNQ TDGTIVWEDD PNRSYTVPAY CGQTTAI |
| gmY_AspaW/537-633 | AVAVTFDLTA TTTYGENIYL VGSISQLGDW DTSDGIALSA DKYTSSNPLW YVTVTLPAGE SFEYKFIRIE SDDSVEWESD PNREYTVPQA CGESTAT |
| gmY_AspniT/537-633 | AVAVTFDLTA TTTYGENIYL VGSISQLGDW ETSDGIALSA DKYTSSDPLW YVTVTLPAGE SFEYKFIRIE SDDSVEWESD PNREYTVPQA CGTSTAT |
| gmY_Neucr/363-460 | TVAVTFNHLA STSYGESIKI VGSISQLGSW SASSGVALSA SQYTTSNPLW TATVSLPAGT KFEYKFVKVS SEGSAVTWES DPNRSYTVPQ SCAESVAV |

18

(continued)

| | |
|---|---|
| 6agtArtgl/864-962 | SVWATFSCEN ATTTFGQSVY VVGNVPQLGN WSPADAVKLE PSAYPTWTGV VRNLPPSSTV EWKCIKRQEA GLPNTADAWE PGGNNILSTP PSGSAGITT |
| 4agtBacFr/149-212 | LAICGNQKAL GNWDPDKAWP MSDANFPEWQ AELDASKLEF PLEYKFVLYN KEEKRAEAWE NNPN |
| 4agtSoltu/8-104 | SRKVSFRIPY YTQWGQNLLI CGSDRLLGSW NVKKGLLLKP SHQGEVLVWS GSIPVPPGYQ SEYSYYVVDD RRNILRWEVG KKRKLLLPDG LQDGQSL |
| 4agt_OrYsa/12-108 | TVTLVFKLPY YTQWGQSLLI AGSEPALGSW NVKQGLSLSP VHQGNELIWS GRVSVATGFT CQYNYYVVDD NKNVLRSESG EKRKLVLPEG VQDGDVV |
| agWdArath/73-155 | KVRLNVRLDH QVNFGDHVAM FGSAKEIGSW KKKSPLNWSE NGWVCELELD GGQVLECKFV IVKNDGSLSW ESGDNRVLKV PNSGNFSVVC HWD |
| genHomsa/263-354 | QVSVRFQVHY VTSTDVQFIA VTGDHECLGR WNTYIPLHYN KDGFWSHSIF LPADTVVEWK FVLVENGGVT RWEECSNRFL ETGHEDKVVH AW |
| laFGalga/2-117 | LFRFGVVLPA RIAEGGGALL VAGSRPELGE WDPQRAVPMQ PARPAAALAA QEPVLWLGEV LLSDEDTASP FWYKFLRREG GQLLWEGNGP HHDRSCVYNQ SNIVDGVYCL PIAHWI |
| laFHomsa/2-130 | RFRFGVVVPP AVAGARPELL VVGSRPELGR WEPRGAVRLR PAGTAAGDGA LALQEPGLWL GEVELAAEEA AQDGAEPGRV DTFWYKFLKR EPGGELSWEG NGPHHDRCCT YNENNLVDGV YCLPIGHWI |
| depChlpr/21-110 | KAKVQFRLPK RVSFGQTISI VTSRSGWEPI PDLHMDWSEG DEWKVSAEVA PGDELEYKYV VLGPSGIVEW QTGSNRRLAV DVPGGAAVTV |
| amyLipko/40-133 | ESVTGSNHVQ LASYEMCGST LSASLYVYND DYDKIVTLYY LTSSGTTGST LALILPVWSN NWELWTLSAI AAGAVEITGA SYVDSDTSVT YTTS |
| amyLipst/40-133 | ESVTSSNHVQ LASHEMCDST LSASLYIYND DYDKIVTLYY LTSSGTTGSV TASYSSSLSN NWELWSLSAP AADAVEITGA SYVDSDASAT YATS |
| gmyArxad/26-132 | NSPPDDKAVA LSSYSYCGGY LSASAFVKNL SYDKLVTLYW TNADNKSTPL NAGSLDYVKA ASDDQSWELW SLNVTTVPDG VDALLNITYV AASIGKTNSQ QLNVQVE |
| gmyRhior/26-130 | ASIPSSASVQ LDSYNYDGST FSGKIYVKNI AYSKKVTVIY ADGSDNWNNN GNTIAASYSA PISGSNYEYW TFSASINGIK EFYIKYEVSG KTYYDNNNSA NYQVS |
| gmyMucci/25-129 | ETVPTTEAVK VKSFTYDGST LAGQIYIKNI AYTKTVTVIY SDASDNWNNN GNTIAASYSA AIAGTNYEYW TFSAPVSGIK QFYVKYVVSG TTYYDNNNSG NYQVS |
| pfHomsa/122-230 | QLQIQKAILE STESLLGSTS IKGIIRVLNV SFEKLVYVRM SLDDWQTHYD ILAEYVPNSC DGETDQFSFK IVLVPPYQKD GSKVEFCIRY ETSVGTFWSN NNGTNYTFI |

EXAMPLE 10 Formation of fibril

The SBD-6H prepared from Example 1 was heated to 97 °C and dropped in room temperature (speed of increasing temperature: 2 °C/ min). The sample was incubated at 37 °C for 2 days. The grid was placed onto sample drops for 30 min containing the thermal induced amyloid-like fibril of SBD-6H. The samples were stained with 4% (w/v) uranyl acetate for 5 min and the grids were then air-fried prior to analysis employing transmission electron microscopy. As shown in Figure 13, fibril of SBD-6H was made.

Alternatively, the amyloid-like fibril formation could be prepared according to the contents disclosed in Yen-Chung Chang and Chin Yu, J. Biol. Chem., Vol. 277, No. 21, Issue of May 24, pp 19027-19036, 2002.

**Sequence list**

<100>GENERAL INFORMATION
<160>NUMBER OF SEQ ID NOS: 3
<200>SEQUENCE CHARACTERISTICS:

<210>SEQUENCE ID NO 1
<211>Length: 117
<212>Type: amino acid
<213>Organism: Rhizopus oryzae Simpson strain
<220>FEATURE
<223>OTHER INFORMATION: starch-binding domain
<400>1

ASIPSSASVQ LDSYNYDGST FSGKIYVKNI AYSKKVTVVY ADGSDNWNNN GNIIAASFSG

PISGSNYEYW TFSASVKGIK EFYIKYEVSG KTYYDNNNSA NYQVSTS

<210>SEQUENCE ID NO 2
<211>Length: 117
<212>Type: amino acid
<213>Organism: Rhizopus oryzae wildtype_strain
<220>FEATURE
<223>OTHER INFORMATION: starch-binding domain
<400>2

ASIPSSASVQ LDSYNYDGST FSGKIYVKNI AYSKKVTVIY ADGSDNWNNN GNTIAASYSA

PISGSNYEYW TFSASINGIK EFYIKYEVSG KTYYDNNNSA NYQVSTS

<210>SEQUENCE ID NO 3
<211>Length: 117
<212>Type: amino acid
<213>Organism: Rhizopus oryzae from U.S. Pat. No. 4863864
<220>FEATURE
<223>OTHER INFORMATION: starch-binding domain
<400>3

ASIPSSASVQ LDSYNYDGST FSGKIYVKNI AYSKKVTVIY ANGSDNWNNN GNTIAASYSA

PISGSNYEYW TFSASINGIK EFYIKYEVSG KTYYDNNNSA NYQVSTS

SEQUENCE LISTING

<110> SIMPSON BIOTECH CO. LTD

<120> RECOMBINANT PROTEIN COMPRISING STARCH BINDING DOMAIN AND USE THEREOF

<130> P2006393C

<150> US 11/070,271

<151> 2005-03-03

<160> 3

<170> PatentIn version 3.2

<210> 1
<211> 107
<212> PRT
<213> Rhizopus oryzae Simpson strain

<220>
<221> starch-binding domain
<222> (1)..(107)

<400> 1

```
Ala Ser Ile Pro Ser Ser Ala Ser Val Gln Leu Asp Ser Tyr Asn Tyr
1               5                   10                  15

Asp Gly Ser Thr Phe Ser Gly Lys Ile Tyr Val Lys Asn Ile Ala Tyr
            20                  25                  30

Ser Lys Lys Val Thr Val Val Tyr Ala Asp Gly Ser Asp Asn Trp Asn
        35                  40                  45

Asn Asn Gly Asn Ile Ile Ala Ala Ser Phe Ser Gly Pro Ile Ser Gly
        50                  55                  60

Ser Asn Tyr Glu Tyr Trp Thr Phe Ser Ala Ser Val Lys Gly Ile Lys
65                  70                  75                  80

Glu Phe Tyr Ile Lys Tyr Glu Val Ser Gly Lys Thr Tyr Tyr Asp Asn
                85                  90                  95

Asn Asn Ser Ala Asn Tyr Gln Val Ser Thr Ser
            100                 105
```

<210> 2
<211> 107
<212> PRT
<213> Rhizopus oryzae wildtype_strain

<220>
<221> starch-binding domain
<222> (1)..(107)

<400> 2

```
Ala Ser Ile Pro Ser Ser Ala Ser Val Gln Leu Asp Ser Tyr Asn Tyr
1               5                   10                  15

Asp Gly Ser Thr Phe Ser Gly Lys Ile Tyr Val Lys Asn Ile Ala Tyr
            20                  25                  30

Ser Lys Lys Val Thr Val Ile Tyr Ala Asp Gly Ser Asp Asn Trp Asn
        35                  40                  45

Asn Asn Gly Asn Thr Ile Ala Ala Ser Tyr Ser Ala Pro Ile Ser Gly
    50                  55                  60

Ser Asn Tyr Glu Tyr Trp Thr Phe Ser Ala Ser Ile Asn Gly Ile Lys
65                  70                  75                  80

Glu Phe Tyr Ile Lys Tyr Glu Val Ser Gly Lys Thr Tyr Tyr Asp Asn
                85                  90                  95

Asn Asn Ser Ala Asn Tyr Gln Val Ser Thr Ser
            100                 105
```

<210> 3
<211> 107
<212> PRT
<213> Rhizopus oryzae from U.S. Pat. No. 4863864

<220>
<221> starch-binding domain
<222> (1)..(107)

<400> 3

```
Ala Ser Ile Pro Ser Ser Ala Ser Val Gln Leu Asp Ser Tyr Asn Tyr
1               5                   10                  15

Asp Gly Ser Thr Phe Ser Gly Lys Ile Tyr Val Lys Asn Ile Ala Tyr
            20                  25                  30

Ser Lys Lys Val Thr Val Ile Tyr Ala Asn Gly Ser Asp Asn Trp Asn
        35                  40                  45

Asn Asn Gly Asn Thr Ile Ala Ala Ser Tyr Ser Ala Pro Ile Ser Gly
    50                  55                  60

Ser Asn Tyr Glu Tyr Trp Thr Phe Ser Ala Ser Ile Asn Gly Ile Lys
65                  70                  75                  80

Glu Phe Tyr Ile Lys Tyr Glu Val Ser Gly Lys Thr Tyr Tyr Asp Asn
                85                  90                  95
```

```
Asn Asn Ser Ala Asn Tyr Gln Val Ser Thr Ser
        100                     105
```

**Claims**

1. A starch binding domain consisting of the amino acid sequence of SEQ ID No. 1.

2. A recombinant protein comprising the starch binding domain as claimed in claim 1 and a polypeptide.

3. An expression vector comprising a gene encoding the starch binding domain as claimed in claim 1.

4. A method for purifying a recombinant protein as claimed in claim 2 comprising:

   (a) applying the biological liquid containing the recombinant protein directly to an affinity matrix;
   (b) eluting the recombinant protein by elution buffer, and
   (c) dialyzing the eluent containing recombinant protein, wherein the affinity matrix is a component containing the formula:

   $$(X\text{-}X)_n$$

   wherein X means glucose molecule, the linkage between glucose and glucose is $\alpha$-1,4- linkage or $\alpha$-1,6-linkage and n is 1 or more than 1, wherein the above formula is contained in a part of the structure selected from a group comprising the main chain, a side chain, or a modified residue of the affinity matrix component.

5. A kit for purifying a recombinant protein comprising
   an expression vector, which comprises a gene encoding the starch binding domain as claimed in claim 1, and
   an affinity matrix to bind the starch binding domain.

6. The kit as claimed in claim 5, which further comprises an elution buffer used to separate the recombinant protein and the affinity matrix.

**Patentansprüche**

1. Stärkebindungsdomäne, die aus der Aminosäuresequenz von SEQ ID Nr. 1 besteht.

2. Rekombinantes Protein, das die Stärkebindungsdomäne nach Anspruch 1 und ein Polypeptid umfasst.

3. Expressionsvektor, der ein Gen umfasst, das die Stärkebindungsdomäne nach Anspruch 1 kodiert.

4. Verfahren zur Aufreinigung eines rekombinanten Proteins nach Anspruch 2, umfassend:

   (a) Auftragen der biologischen Flüssigkeit, die das rekombinante Protein enthält, direkt auf eine Affinitätsmatrix,
   (b) Eluieren des rekombinanten Proteins durch einen Elutionspuffer, und
   (c) Dialyse des Eluenten, der das rekombinante Protein enthält, wobei die Affinitätsmatrix eine Komponente ist, die die Formel

   $$(X\text{-}X)_n$$

   umfasst, wobei X ein Glukosemolekül bezeichnet, wobei die Verknüpfung zwischen Glukose und Glukose eine $\alpha$-1,4- Verknüpfung oder eine $\alpha$-1,6 Verknüpfung ist und n gleich 1 oder größer als 1 ist, wobei die vorstehende Formel in einem Teil einer Struktur enthalten ist, die ausgewählt ist aus einer Gruppe, die die Hauptkette, eine Seitenkette oder einen modifizierten Rest der Affinitätsmatrix-Komponente umfasst.

5. Set zur Aufreinigung eines rekombinanten Proteins, umfassend

einen Expressionsvektor, der ein Gen umfasst, das die Stärkebindungsdomäne nach Anspruch 1 kodiert, und eine Affinitätsmatrix zur Bindung der Stärkebindungsdomäne.

6.  Set nach Anspruch 5, das ferner einen Elutionspuffer umfasst, der zur Trennung des rekombinanten Proteins und der Affinitätsmatrix verwendet wird.


**Revendications**

1.  Domaine de liaison à l'amidon constitué de la séquence d'acides aminés de SEQ ID No. 1.

2.  Protéine recombinée comprenant le domaine de liaison à l'amidon selon la revendication 1 et un polypeptide.

3.  Vecteur d'expression comprenant un gène codant pour le domaine de liaison à l'amidon selon la revendication 1.

4.  Procédé de purification d'une protéine recombinée selon la revendication 2, comprenant:

    (a) l'application du liquide biologique contenant la protéine recombinée directement sur une matrice d'affinité;
    (b) l'élution de la protéine recombinée par un tampon d'élution, et
    (c) la dialyse de l'éluant contenant la protéine recombinée, dans lequel la matrice d'affinité est un composant contenant la formule:

$$(X\text{-}X)_n$$

    où X désigne une molécule de glucose, la liaison entre glucose et glucose est une liaison $\alpha$-1,4 ou une liaison $\alpha$-1,6 et n vaut 1 ou plus de 1, dans lequel la formule ci-dessus est contenue dans une partie de la structure choisie dans un groupe comprenant la chaîne principale, une chaîne latérale ou un résidu modifié du composant de matrice d'affinité.

5.  Trousse pour la purification d'une protéine recombinée comprenant
    un vecteur d'expression, qui comprend un gène codant pour le domaine de liaison à l'amidon selon la revendication 1, et
    une matrice d'affinité pour lier le domaine de liaison à l'amidon.

6.  Trousse selon la revendication 5, qui comprend en outre un tampon d'élution utilisé pour séparer la protéine recombinée et la matrice d'affinité.

Figure 1

Figure 2

Figure 3

**Association rate assay**

Figure 4

**wt SBD**

$B_{max}=$ 41.14 $\mu$mol/g
$K_d=$ 1.43 $\mu$M

Figure 5

Figure 6

**Figure 7**

**A**

RoGACBM21
MdGACBM21
AaGACBM21
LkaACBM21
HsPPCBM21
AnGACBM20

**B**

N β Y β Y β W β Y β N β N β Y β N

RoGACBM21
AnGACBM20

N β Y β W β Y β N β N β N β N β N

**C**

RoGACBM21          AnGACBM20          MdGACBM21          AaGACBM21

LkaACBM21          HsPPCBM21

Figure 8

Figure 9

Figure 10

Figure 11

Figure 12

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5643758 A
- US 5202247 A
- EP 0186066 A1
- WO 0077165 A2
- US 4863864 A
- US 11070271 B

### Non-patent literature cited in the description

- **BORASTON et al.** *Biochem. J.,* 2004, vol. 382, 769-781
- **FANG et al.** *Protein Engineering,* 1998, vol. 11, 119-126
- **SAUER et al.** *Biochemistry,* 2001, vol. 40, 9336-9346
- **BOLAM et al.** *Biochem. J.,* 1998, vol. 331, 775-781
- **CHARONCK et al.** *Biochemistry,* 2000, vol. 39, 5013-5021
- **ALI et al.** *Biosci. Biotechnol. Biochem.,* 2001, vol. 65, 41-47
- **HOUGHTON-LARSEN et al.** *Appl. Microbiol. Biotechnol.,* 2003, vol. 62, 210-217
- **PALDI et al.** *Biochem. J.,* 2003, vol. 372, 905-910
- **BELSHAW et al.** *FEBS Lett.,* 1990, vol. 269, 350-353
- **J. A., CLAMP ; M. E., SIDDIQUI ; A. S., FINLAY, M. ; BARTON, G. J.** JPred: a consensus secondary structure prediction server. *Bioinformatics,* 1998, vol. 14, 892-893
- **COMBET, C. ; BLANCHET, C. ; GEOURJON, C. ; DELEAGE, G.** NPS@: network protein sequence analysis. *Trends Biochem. Sci.,* 2000, vol. 25, 147-150
- **THOMPSON, J. D. ; GIBSON, T. J. ; PLEWNIAK, F. ; JEANMOUGIN, F. ; HIGGINS, D. G.** The CLUSTAL_X windows interface: flexible strategies for multiple sequence alignment aided by quality analysis tools. *Nucleic Acids Res.,* 1997, vol. 25, 4876-4882
- **SCHWEDE, T. ; KOPP, J. ; GUEX, N. ; PEITSCH, M. C.** SWISS-MODEL: An automated protein homology-modeling server. *Nucleic Acids Res.,* 2003, vol. 31, 3381-3385
- **SORIMACHI, K. et al.** Solution structure of the granular starch binding domain of glucoamylase from Aspergillus niger by nuclear magnetic resonance spectroscopy. *J. Mol. Biol.,* 1996, vol. 259, 970-987
- **PEARL, F.** The CATH Domain Structure Database and related resources Gene3D and DHS provide comprehensive domain family information for genome analysis. *Nucleic Acids Res.,* 2005, vol. 33, D247-251
- **ROBERGE, M. ; LEWIS, R. N. ; SHARECK, F. ; MOROSOLI, R. ; KLUEPFEL, D. ; DUPONT, C. ; MCELHANEY, R. N.** Differential scanning calorimetric, circular dichroism, and Fourier transform infrared spectroscopic characterization of the thermal unfolding of xylanase A from Streptomyces lividans. *Proteins,* 2003, vol. 50, 341-354
- **SPANDE, T. F. ; WITKOP, B.** Determination of the tryptophan content of proteins with N-bromosuccinimide. *Methods Enzymol.,* 1967, vol. 11, 498-506
- **SOKOLOVSKY et al.** Tetranitromethane. A reagent for the nitration of tyrosyl residues in proteins. *Biochemistry,* 1966, vol. 5, 3582-3589
- **BRAY, M. R. ; CARRIERE, A. D. ; CLARKE, A. J.** Quantitation of tryptophan and tyrosine residues in proteins by fourth-derivative spectroscopy. *Anal. Biochem.,* 1994, vol. 221, 278-284
- **PALDI, T. et al.** Glucoamylase starch-binding domain of Aspergillus niger B1: molecular cloning and functional characterization. *Biochem. J.,* 2003, vol. 372, 905-910
- **BELSHAW, N. J.** Production and purification of a granular-starch-binding domain of glucoamylase 1 from Aspergillus niger. *FEBS Lett.,* 1990, vol. 269, 350-353
- **WILLIAMSON, M. P. et al.** Function of conserved tryptophans in the Aspergillus niger glucoamylase 1 starch binding domain. *Biochemistry,* 1997, vol. 36, 7535-7539
- **GIARDINA, T. et al.** Both binding sites of the starch-binding domain of Aspergillus niger glucoamylase are essential for inducing a conformational change in amylose. *J. Mol. Biol.,* 2001, vol. 313, 1149-1159
- **YEN-CHUNG CHANG ; CHIN YU.** *J. Biol. Chem.,* 24 May 2002, vol. 277 (21), 19027-19036